Europäisches Patentamt

European Patent Office

Office européen des brevets

(19)

(11) Publication number: **0 195 285**
**A2**

(12) # EUROPEAN PATENT APPLICATION

(21) Application number: 86102547.6

(22) Date of filing: 27.02.86

(51) Int. Cl.⁴: **C 12 N 15/00**
C 12 P 21/02, A 01 N 63/00
C 12 Q 1/68, C 12 N 1/20
//(C12N1/20, C12R1:07)

A request for correction of figure 8 has been filed pursuant to Rule 88 EPC. A decision on that request will be taken by the Examining Division.

(30) Priority: 28.02.85 US 706836

(43) Date of publication of application:
24.09.86 Bulletin 86/39

(84) Designated Contracting States:
AT BE CH DE FR GB IT LI LU NL SE

(71) Applicant: UNIVERSITY OF GEORGIA RESEARCH
FOUNDATION, INC.
612 Boyd Graduate Studies Research Center
Athens, GA 30602(US)

(72) Inventor: Sekar, Vaithilingam, Sekar
245 North Thompson Drive Apt. N. 7
Madison Wisconsin 53714(US)

(72) Inventor: Carlton, Bruce D.
1385 Eagle Road
New Hope Pennsylvania 18938(US)

(74) Representative: Giambrocono, Alfonso, Dr. Ing. et al,
Ing. A. Giambrocono & C. S.r.l. Via Rosolino Pilo 19/B
I-20129 Milano(IT)

(54) Molecular cloning of the delta-endotoxin gene of bacillus thurigiensis var. israelensis.

(57) A recombinant vector capable of replicating in a microorganism host, in which the vector contains expressible heterologous genetic information coding for a protein having toxic properties of δ-endotoxin from *Bacillus thuringiensis* var. *israelensis*, is disclosed along with isolated DNA and RNA coding for the protein, transformed microorganisms containing the vector, the toxic protein itself, and methods of using the toxic protein as an insecticide.

EP 0 195 285 A2

## Description

## Molecular Cloning of the δ-Endotoxin Gene of Bacillus Thuringiensis Var. Israelensis

### Technical Field

The present invention relates to the δ-endotoxin
of Bacillus thuringiensis var. israelensis, a variety
of Bacillus thuringiensis whose crystal protein has
been shown to be toxic to aquatic forms of dipteran
insects. More particularly, the invention relates to
improved means for producing such toxic proteins using
techniques of genetic engineering.

### Background Art

Bacillus thuringiensis (BT) is a gram-positive,
sporeforming soil bacterium which has the ability to
produce phase-refractile, generally bipyramidal,
proteinaceous, crystalline inclusions during sporulation [Bulla, L.A., Jr., Kramer, K.J. and Davidson,
L.I.: "Characterization of the entomocidal parasporal
crystal of Bacillus thuringiensis." J. Bacteriol. 130
(1977) 375-381]. These crystal proteins (termed
δ-endotoxins) are toxic to larvae of many lepidopterous
insects [Dulmage, H.T.: "Genetic Manipulation of
Pathogens: Selection of Different Strains," in Hoy,
M.A. and McKelvey, J.J., (Eds.) Genetics in Relation to
Insect Management, (1979) pp. 116-127, Rockefeller
Foundation, New York]. To date, over 20 varieties of
BT have been described and classified according to
their flagellar immunological serotypes [de Barjac, H.
and Bonnefoi, A.: "Mise au Point sur la Classification

des <u>Bacillus thuringiensis</u>," <u>Entomophaga</u> <u>18</u> (1973) 5-17].

One such variety, <u>B</u>. <u>thuringiensis</u> var. <u>israelensis</u> (BTI), isolated in Israel in 1977, differs from the other varieties in that its parasporal inclusions are irregular in shape and are highly toxic to larvae of several dipteran aquatic insects, such as mosquitos and black flies [Goldberg, L.J. and Margalit, J.: "A Bacterial Spore Demonstrating Rapid Larvicidal Activity against <u>Anopheles sergentii</u>, <u>Uranatenia unguiculata</u>, <u>Culex univittatus</u>, <u>Aedes aegypti</u> and <u>Culex pipiens</u>," <u>Mosquito News</u> <u>37</u> (1977) 353-358]. Due to its unique insecticidal property, <u>BTI</u> δ-endotoxin has an important place in the control of several human disease vectors in many parts of the world.

The recent development of techniques of genetic engineering has lead to the production of many substances of biological origin in microorganisms which do not normally produce these substances. Genetic engineering, based generally on the techniques set forth in Cohen and Boyer, U.S. Patent 4,237,224, has now reached full development as a technology in only a few years. Indeed, the genetic engineering of <u>Escherichia coli</u> to produce <u>Bacillus thuringiensis</u> var. <u>kurstaki</u> crystal protein is reported in U.S. Patent 4,448,885 to Schnepf and Whiteley. In this patent, expression of the crystalline protein is achieved <u>in E. coli</u> by use of plasmids containing heterologous DNA coding for the <u>BT</u> crystal protein. A partial equivalent of this U.S. Patent is European Patent 63949. European Patent Appl. 93062-A1 describes cloning of a crystal protein gene obtained from <u>Bacillus thuringiensis</u> var. <u>berliner</u> in a similar manner.

However, the characteristics of other BT crystal proteins are different from those of the BTI crystal protein. Other BT crystal proteins are not effective against aquatic larvae of mosquitos and other dipteran insects as is the crystal protein of BTI. Accordingly, because of the toxic effects of the crystal protein of BTI on mosquitos and blackflies and because of the prevalence of mosquitos and blackflies as disease vectors, there is a need for increased availability of BTI crystal protein.

## Disclosure of the Invention

Accordingly, it is an object of this invention to provide a recombinant DNA plasmid capable of expressing a protein having the toxic effects to insect pests of BTI crystal protein.

It is another object of this invention to provide an isolated DNA sequence useful in the production of such a recombinant plasmid.

It is a further object of this invention to provide a combination of recombinant plasmid and host in which BTI crystal protein is expressed with high efficiency.

It is yet another object of this invention to provide a protein having reduced toxicity to mammals while retaining BTI-like effectiveness against insects.

These and other objects of the invention as will hereinafter become more readily apparent have been accomplished by providing a recombinant DNA plasmid capable of replicating in a bacterial host, said

0195285

plasmid containing expressable heterologous DNA coding for a toxic protein having a toxic property of δ-endotoxin from <u>Bacillus thuringiensis</u> var. <u>israelensis</u>. DNA molecules, transformed microorganisms, and proteins having toxic properties similar to <u>BTI</u> endotoxin but having reduced mammalian toxicity are also provided by this invention.

## Brief Description of the Drawings

A more complete appreciation of the invention and many of the attendant advantages thereof will be readily obtained by reference to the following detailed description when considered in connection with the accompanying drawings, wherein:

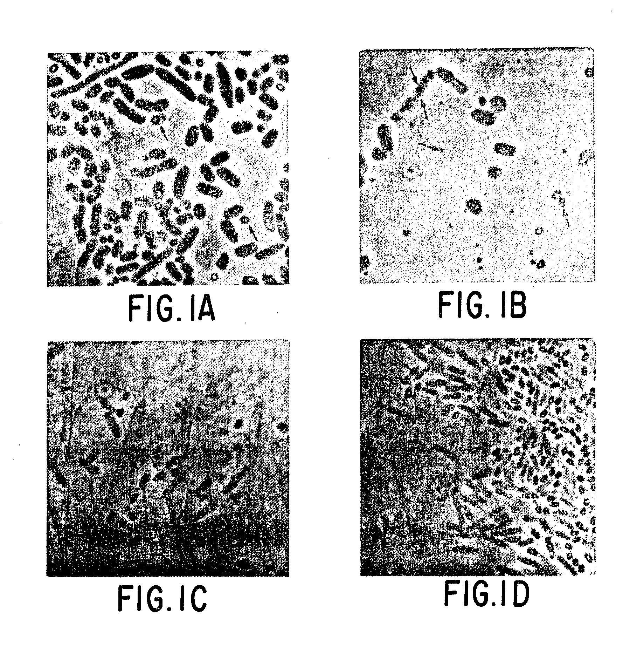

FIGURE 1 shows phase-contrast photomicrographs of sporulating <u>B. megaterium</u> and <u>B. subtilis</u> cells showing production of crystal inclusions by recombinant clones. (A) <u>B. megaterium</u> transformed with pBC16 vector plasmid. (B) <u>B. megaterium</u> recombinant clone VB131. (C) <u>B. subtilis</u> strain SB131. Spores are indicated by solid arrows (———>) and crystal inclusions by dashed arrows (- - ->).

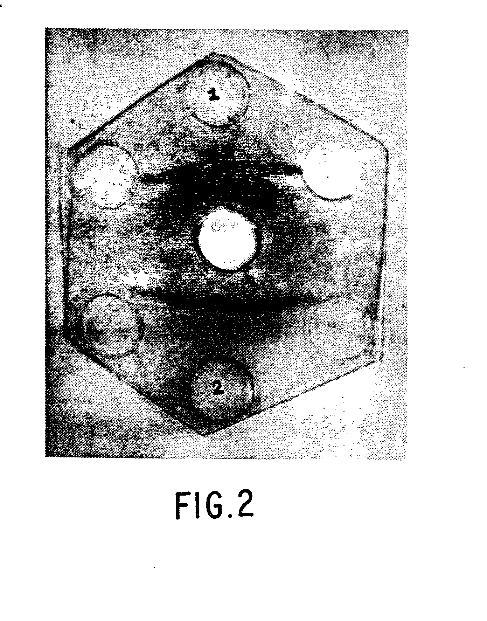

FIGURE 2 shows a photograph illustrating detection of the crystal antigen in <u>B. megaterium</u> clone VB131 by double immunodiffusion. Rabbit antiserum to natural <u>BTI</u> crystal protein was placed in the center well. Well 1: solubilized crystal protein (~5 µg) from <u>BTI</u>; well 2: solubilized crystal protein (~5 µg) from <u>B. megaterium</u> VB131.

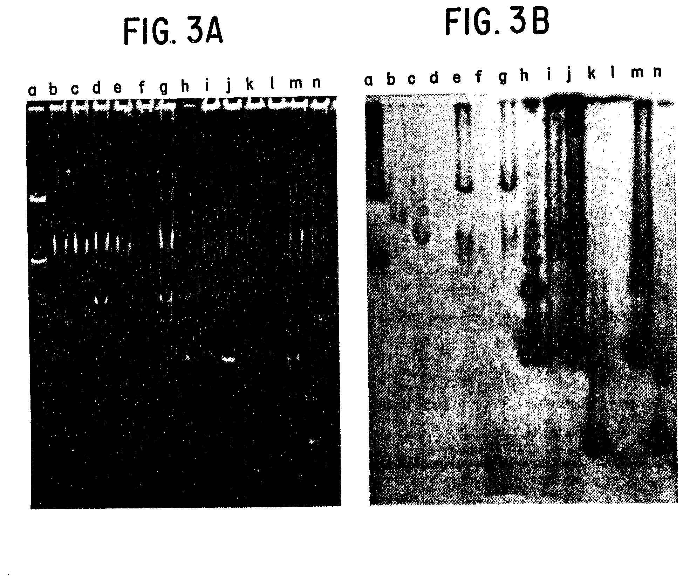

FIGURE 3 shows photographs illustrating agarose gel and Southern blotting analysis of various plasmid

0195285

DNAs. Plasmid DNAs of several BTI, B. megaterium and B. subtilis strains, along with purified 112-kb and pVB131 plasmid DNAs, were separated on a 0.5% agarose gel (Panel A). After transferring to a nitrocellulose filter, the plasmids were probed with $^{32}$P-labeled pVB131 DNA (Panel B). (a) 112-kb plasmid DNA; (b) HD567-40; (c) HD567-39; (d) HD567-42; (e) HD567-61-9; (f) HD567-61; (g) HD567-1; (h) VB131 plasmid DNA; (i) B. megaterium VB381 (a Cry$^-$ recombinant carrying a 12-kb plasmid); (j) B. megaterium VB131; (k) B. megaterium VT1650; (1) B. megaterium VT1660; (m) B. subtilis SB131; (n) B. subtilis MI112 transformed with pBC16.

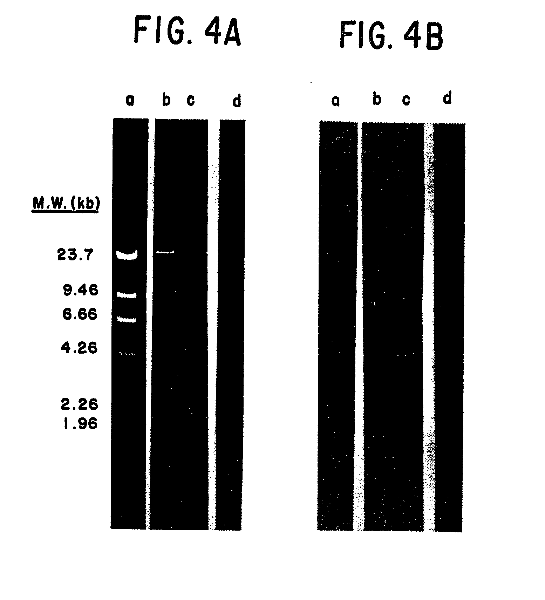

FIGURE 4 shows photographs illustrating agarose gel and Southern blotting analysis of restriction digests of toxin plasmid DNAs. Restriction fragments of XbaI-digested 112-kb and pVB131 DNAs were separated on a 0.5% agarose gel (Panel A) and analyzed by Southern blot hybridization (Panel B), using as a probe $^{32}$P-labeled pVB131 DNA. The sizes (in kb) of HindIII digested phage λ DNA restriction fragments (lane a) are listed on the left margin; (b) 112-kb plasmid DNA digested with XbaI; (c) pVB131 DNA digested with XbaI; (d) undigested 112-kb plasmid DNA.

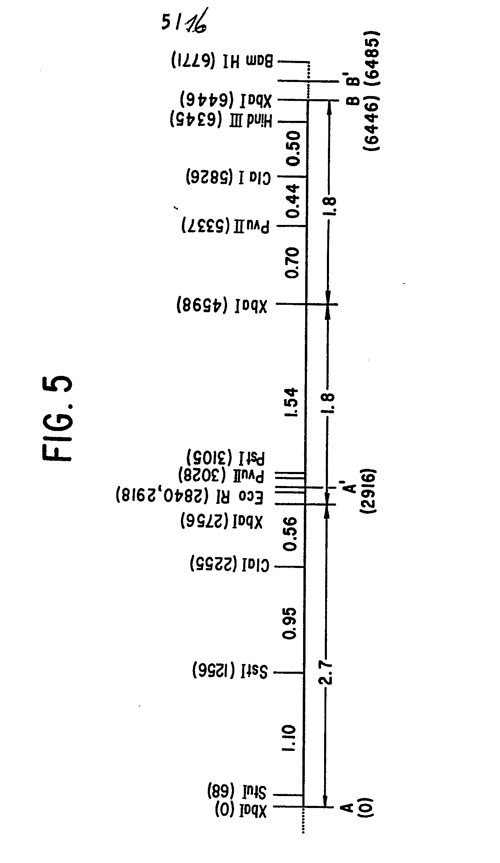

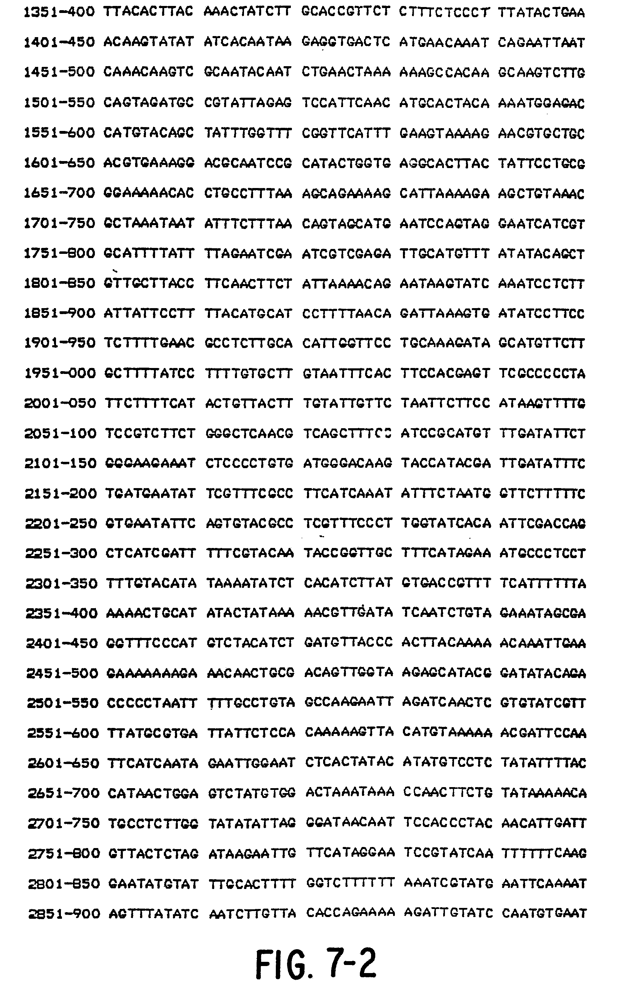

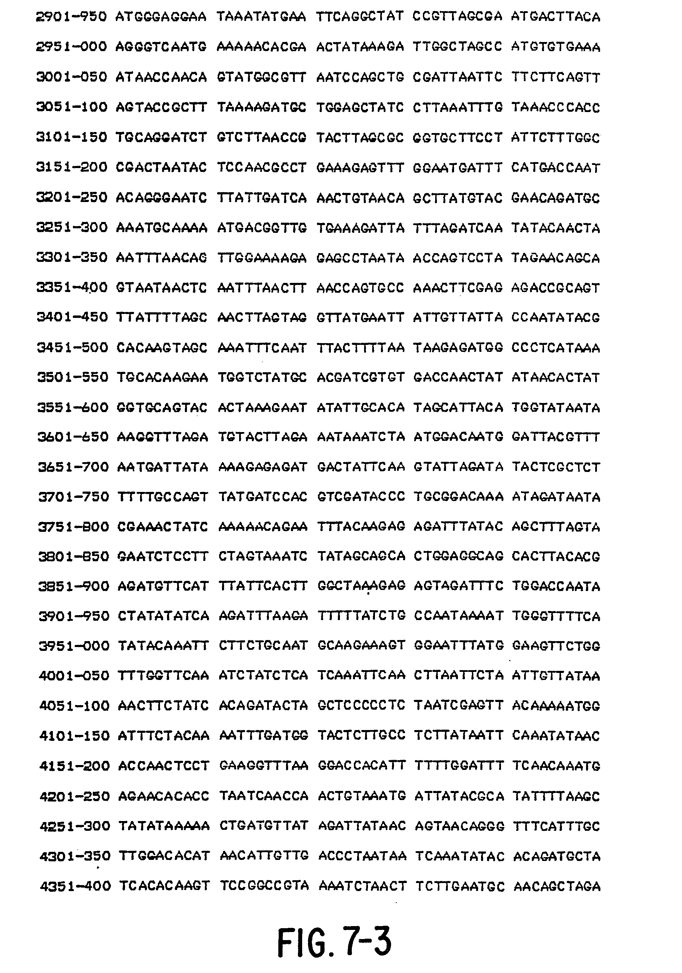

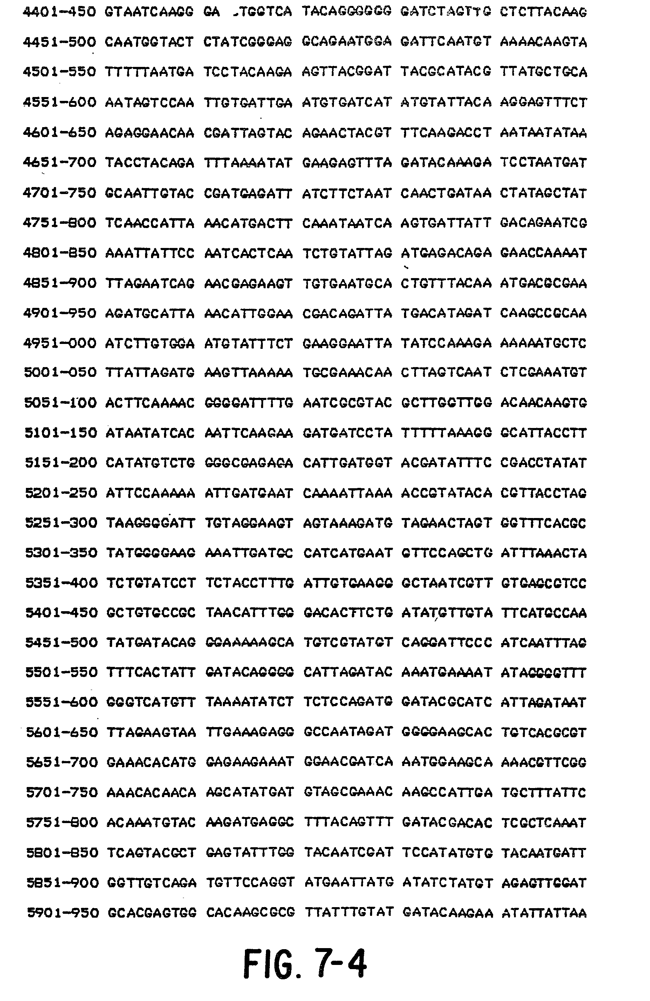

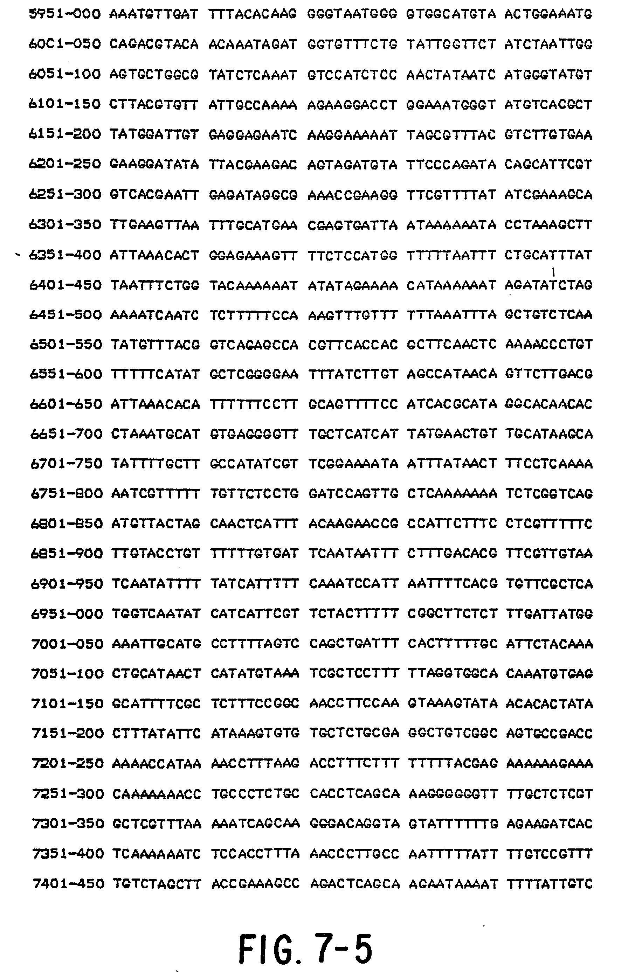

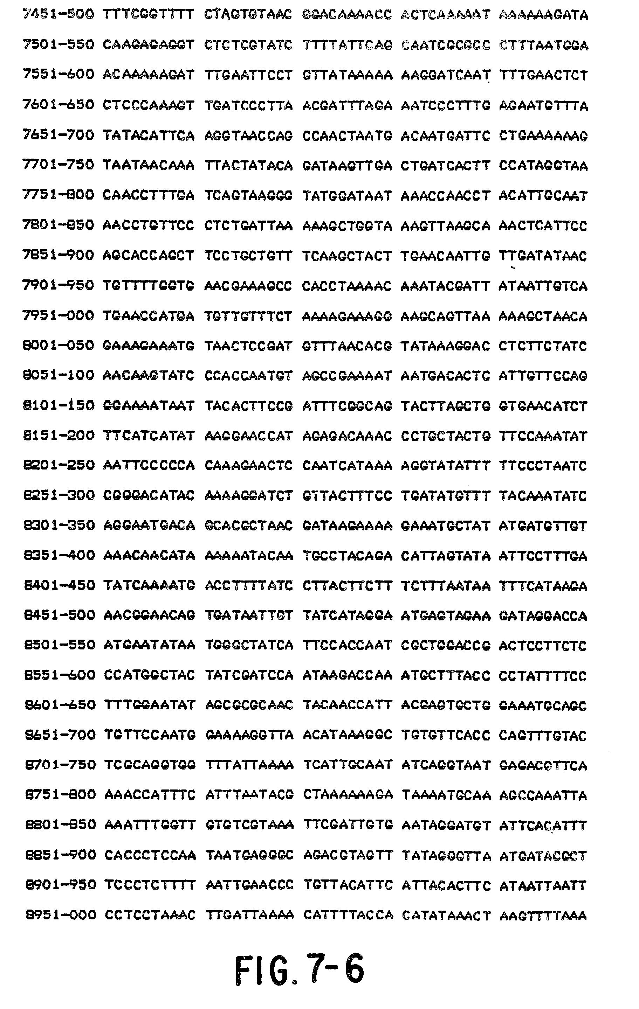

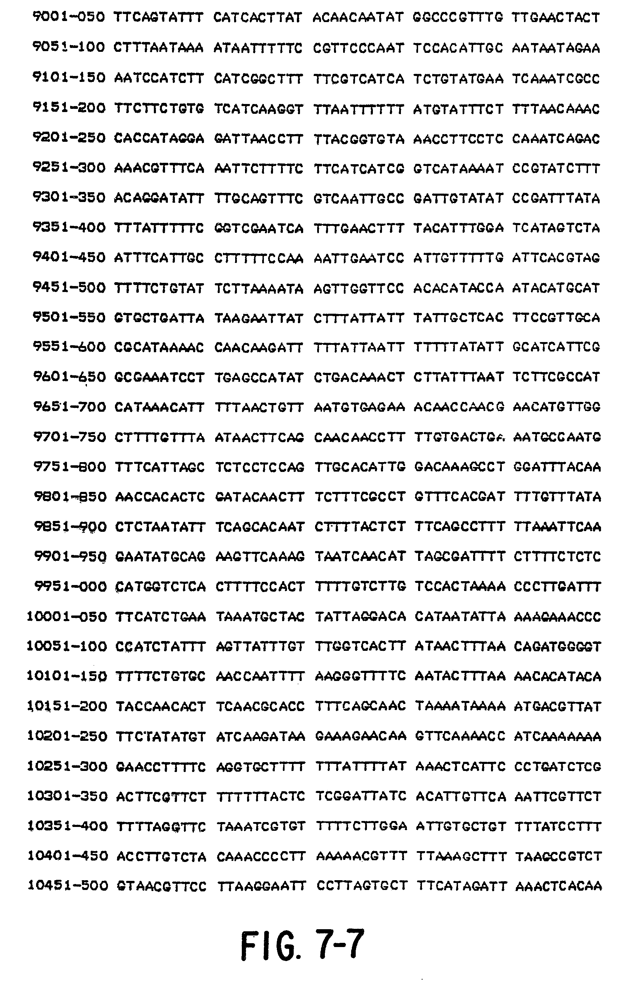

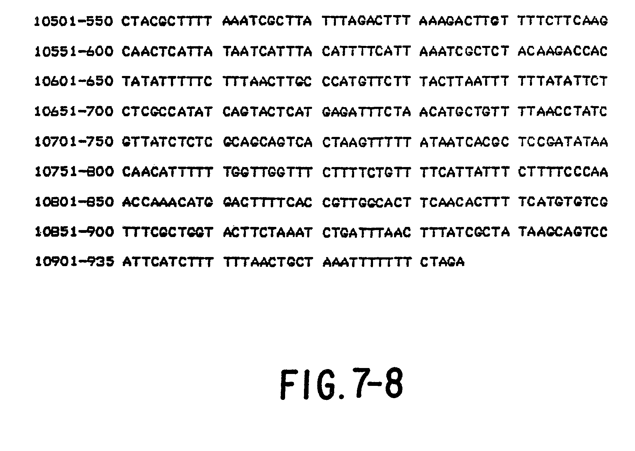

FIGURE 5 shows in schematic form a restriction map of the cloned XbaI insert from the BTI 112-kb toxin plasmid containing a segment of the δ-endotoxin gene. The relative locations of various restriction cleavage sites, determined as described in the Example under Materials and Methods, Section (i), are shown parallel to the line representing the DNA chain. Sizes of fragments are given in kb. The three XbaI fragments of the cloned DNA insert and their sizes (in kb) are shown below the restriction map. The dotted regions represent a part of the pBCl6 vector DNA. Numbers in parenthesis show locations as determined from the sequenced gene shown in FIGURE 7. A and A' are start locations for the BTI segment and expressed protein, respectively, while B and B' are the corresponding stop locations.

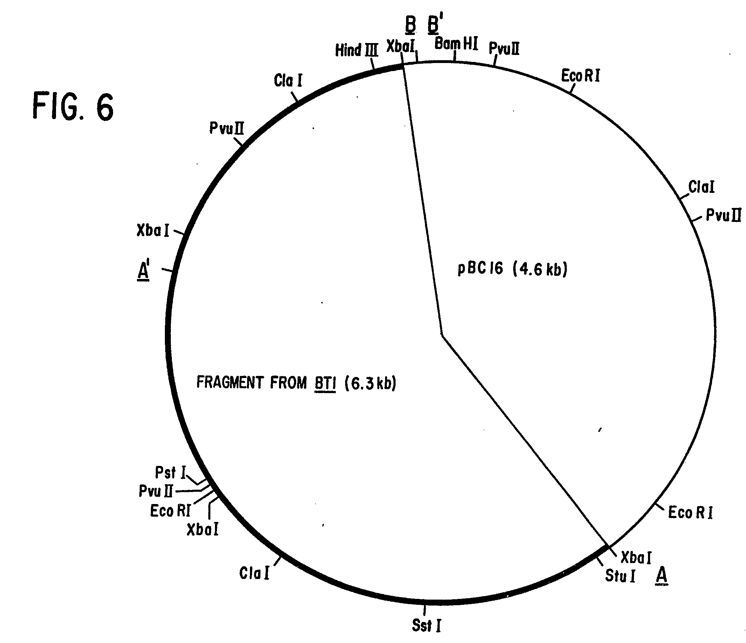

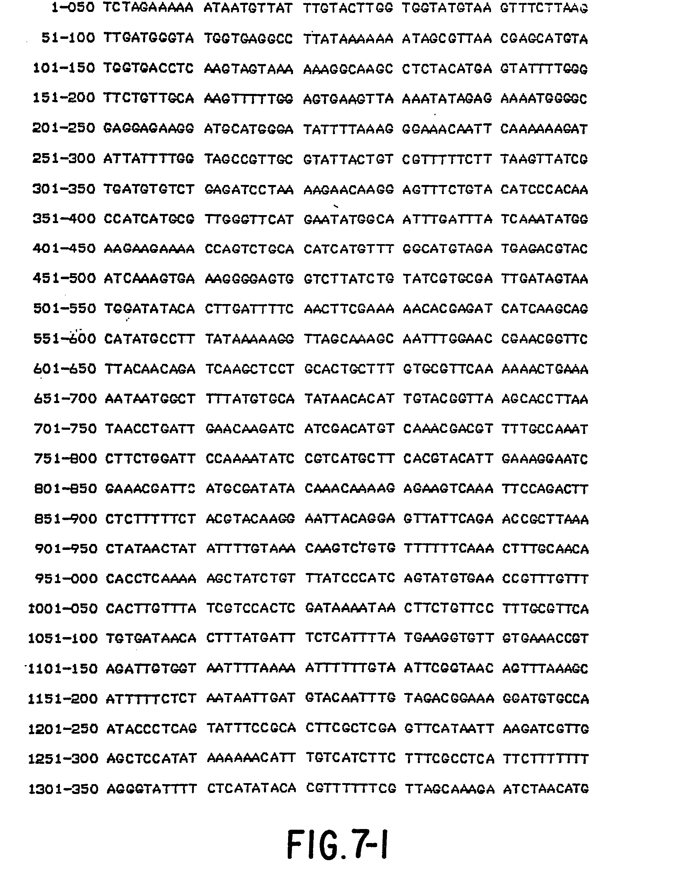

FIGURE 6 is a restriction endonuclease cleavage map of the VB131 clone plasmid showing the pBCl6 host vector of 4.5-kb (as the lighter circular arc B clockwise to A) and the 6.3-kb fragment originating from Bacillus thuringiensis var. israelensis (as the heavier circular arc A clockwise to B). The DNA sequence for a gene segment coding for toxic protein is shown as the portion of the heavier circular arc reading A' clockwise to B (segment from BTI) or A' clockwise to B'(entire epressed toxic protein).

FIGURE 7 (sheets 7-1 to 7-8) shows the complete base sequence for the DNA of VB131 clone circular plasmid obtained from the Bacillus thuringiensis var. israelensis. This base sequence starts at the 4 o'clock point of FIGURE 6, which corresponds to the heavier circular arc clockwise from A. Promoter regions are found at 2833 through 2844 and 2854 through 2894. A ribosome binding site (GGGAGG) is found at 2904 through 2909. A start signal (ATGAATT) is found at 2916 through 2922. A stop codon (TAA) is found at 6483 through 6485. XBA-I sites are at 0001, 2756, 4588 and 6446, respectively. The portion of this base sequence from section 2916 to 6485 is the base sequence for a DNA fragment coding for the expressed toxic protein corresponding to that portion of the arc reading clockwise from A' to B' in FIGURE 6.

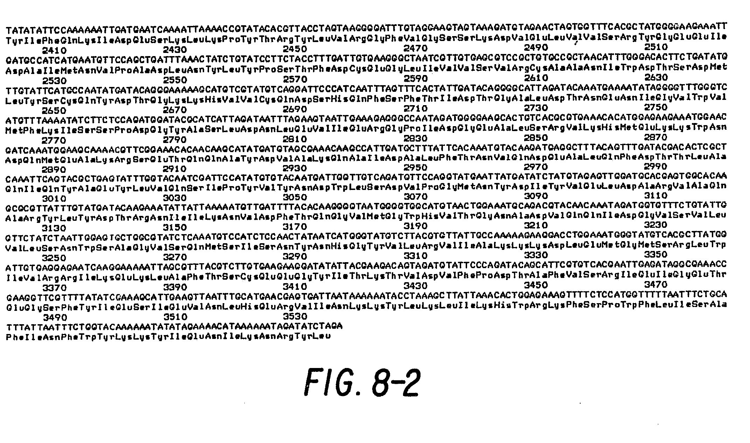

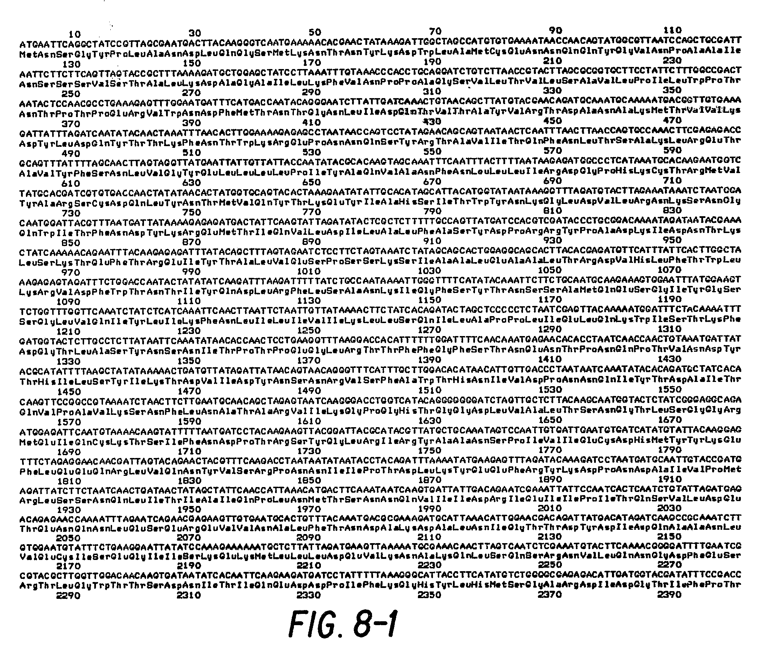

FIGURE 8 (sheets 8-1 to 8-2) shows the deduced toxic protein amino acid sequence coded by the sequence of the gene of VB131 clone obtained from Bacillus thuringiensis var. israelensis set forth in FIGURE 7 (from 2916 to 6446).

## Best Mode for Carrying Out the Invention

Plasmid-curing and plasmid-transfer experiments have shown that a 112-kb plasmid (sometimes referred to as a 75-megadalton plasmid) is associated with crystal toxin production in BTI. The 112-kb plasmid was used as a gene source for the BTI δ-endotoxin gene cloned by the present inventors. Development of an efficient protoplast transformation system for B. megaterium in the inventors' laboratory assisted in the cloning of the δ-endotoxin gene of BTI in B. megaterium. In contrast, hosts such as Escherichia coli or B. subtilis had been used for the cloning of the crystal toxin gene of other varieties of BT by other investigators [Schnepf, H.E. and Whiteley, H.R.: "Cloning and Expression of the Bacillus thuringiensis Crystal Protein Gene in Escherichia coli," Proc. Natl. Acad. Sci. USA 78 (1981) 2893-2897; Kronstad, J.W., Schnepf, H.E. and Whiteley, H.R.: "Diversity of Locations for Bacillus thuringiensis Crystal Protein Genes," J. Bacteriol. 154 (1983) 419-428; Held, G.A., Bulla, L.A., Jr., Ferrari, E., Hoch, J., Aronson, A.I. and Minnich, S.A.: "Cloning and Localization of the Lepidopteran Protoxin Gene of Bacillus thuringiensis subsp. kurstaki," Proc. Natl. Acad. Sci USA 79 (1982) 6065-6069; Klier, A., Fargette, F., Ribier, J. and Rapoport, G.: "Cloning and Expression of the Crystal Protein Genes from Bacillus thuringiensis strain berliner 1715," EMBO Journal 1 (1982) 791-799].

The molecular cloning of the δ-endotoxin gene of BTI in a B. megaterium host was one of the events that gave rise to the present invention. A segment of DNA from the 112-kb plasmid isolated from BTI (described later in more detail) has been isolated and has been identified as a source of DNA coding for a crystalline protein having immunological cross-reactivity with crystal protein of BTI. The protein produced by a plasmid containing this isolated DNA segment exhibits toxicity against mosquito larvae but lacks toxicity against mammalian cells (demonstrated using suckling mice). Furthermore, now that a DNA segment from a gene coding for the BTI δ-endotoxin has been identified, it is readily possible to isolate variations of this protein through known techniques of genetic engineering such as site-specific mutagenesis and DNA hybridization. Genes similar to but slightly different from the gene segment that has been isolated can readily be identified by hybridizing a DNA material from other BTI stains with radioactively labeled DNA segments from the now-identified BTI δ-endotoxin gene. Evidence to date indicates that all existing BTI strains were derived from a single original isolate.

The following technique, which is described for purposes of illustration only and is not intended to be limiting, provides a method for identifying genetic material that codes for BTI δ-endotoxin and proteins derived therefrom or otherwise related thereto. The 6.3-kb segment of DNA obtained by the use of XbaI restriction endonucleases from the 112-kb plasmid of BTI (or any other source as described herein) is labeled with radioactive phosphorus using known techniques. Alternatively, smaller sections of the 6.3-kb segment can be obtained (for example by using any of the restriction endonucleases identified in this application to cleave the segment) and labeled with radioactive phosphorus. The labeled DNA is used as a hybridization probe to identify complementary DNA from other sources. Several suitable hybridization techniques are described in detail later in this application. DNA is isolated from a potential source of BTI δ-endotoxin genes (typically a variety of BTI obtained by mutation or a wild type obtained from another source) using standard techniques of DNA purification. Typically, the DNA being hybridized with the probe is obtained by cleavage of the isolated DNA with restriction endonucleases or chemical means. When a gene segment has been identified by its ability to hybridize with a labeled probe, the identified segment is inserted into a plasmid capable of replicating in a bacterial host. Techniques of inserting heterologous DNA into a plasmid in a manner in which the DNA can be expressed (i.e., inserting the DNA downstream from a promoter and in reading frame) are well known in the art.

The δ-endotoxin of BTI was identified in the inventors' laboratories to be associated with a 112-kb plasmid. On digestion with XbaI restriction endonuclease, the 112-kb plasmid yields (among 20-plus fragments) a 2.7-kb segment and two 1.8-kb segments. These three segments combine together on ligation to give a 6.3-kb segment which contains a sequential series of restriction endonuclease sites comprising XbaI, StuI, SstI, ClaI, XbaI, EcoRI, PvuII, PstI, XbaI, PvuII, ClaI, HindIII, and XbaI. The restriction map showing the order of cleavage and the distance (in kilobases) between cleavage sites is set forth in Figure 5. A DNA segment coding for a toxic protein having a toxic property of BTI δ-endotoxin can readily be isolated by known techniques of genetic engineering, given the information set forth herein, from any strain or variety of BTI. This is particularly true in the present situation since all known strains of BTI are believed to be derived from a single strain and to be closely related genetically.

The term "segment" is used in this application to describe a section of DNA, RNA, or a protein that forms or is identical to part of a larger molecule. The term is defined further by the context in which it is found. For example, the phrase "a segment of DNA coding for all or part of a BTI gene" includes segments completely within the expressible sequence of such a gene, segments containing terminal portions of the expressible sequence along with non-expressed condons, and segments that encompass the entire BTI gene (optionally including stop codons, non-expressed segments, promoter sites, and the like).

Several terms of different scope are used to name the proteinaceous compounds of the invention. The broadest of these is "toxic protein" by which is meant a protein having toxicity against the aquatic larvae of a dipteran insect, whether that protein is currently in crystalline form or is crystalizable, i.e., capable of forming crystals of the BTI type. The terms "endotoxin" and "crystal protein" are sometimes used synonymously with the term "toxic protein". The terms " δ-endotoxin" and "BTI crystal protein" are more specific terms referring to the natural products produced by wild or mutated strains of BTI and to genetically engineered products that are identical or substantially identical to the natural products, as indicated by the context of the term.

The toxic proteins of this application are characterized by having a toxic property in common with a BTI δ-endotoxin. By "toxic property" is meant ability to kill or otherwise hinder growth and surfival of any insect pest that is affected by a BTI δ- endotoxin. A preferred toxic property is toxicity to Dipteran insects, especially mosquito larvae. The term "toxic" means that a measurable fraction (preferably at least 20%, more preferably at least 50%) of a population of larvae is killed when the protein is applied in any manner described in this application (infra). A toxic protein of the invention need not exhibit the same degree of toxicity as a BTI δ-endotoxin to which it is being compared but may show either lesser or greater toxicity.

One preferred embodiment of the invention, discussed later in detail, is a toxic protein whose amino-terminus portion is identical or substantially identical to the amino portion, but whose carboxy-terminus portion is different from the normal carboxy-terminus portion, of a δ-endotoxin. Such proteins are produced, for example, by using a segment of BTI DNA containing the start codon and initial segment of a δ-endotoxin gene but terminating at a restriction site prior to the normal termination codon of the δ-endotoxin gene. When such partial δ- endotoxin genes are expressed, a composite protein is formed in which the amino-terminus portion is formed from the δ-endotoxin gene (start codon to clearage site) and the carboxy-terminus portion is formed from the host vector codons [cleavage site to first in-frame stop codon]. Thus the terms "amino-terminus portion" and "carboxy-terminus portion" separately refer to the two ends of the toxic protein without reference to length (unless specified) and together constitute the entire toxic peptide amino acid sequence.

Proteinaceous and polynucleotide products of the invention will generally contain a segment identical or substantially identical to a segment of a corresponding natural product. By "substantially identical" is meant, when referring to a polynucleotide, that detectable hybridization between the corresponding segments will take place (or that the segment is genetically equivalent, i.e., codes for the same amino acids, to a segment hybridizable with a natural product segment) and, when referring to a protein or peptide, that at least 80%, preferably 90%, and more preferably 95%, of the amino acids are identical. Identity, whether complete or substantial, is preferably measured over a segment consisting of at least 5 amino acids (more preferably at least 10 and most preferably at least 20) when proteins or peptides are being compared and over at least 10 nucleotides (more preferably at least 15 and most preferably at least 20) when DNA or RNA molecules are being compared by hybridization. Greater segment lengths that exhibit hybridization with a segment of an actual δ-endotoxin gene and range in size up to the full length of the δ-endotoxin gene segments identified in this specification are also preferred. Such materials generally have toxic properties that more closely resemble those of the natural δ-endotoxin, but smaller segments are also useful as hybridization probes or precursors thereof.

When a segment of genetic information is said to be hybridizable with a <u>Bacillus thuringiensis</u> var.

israelensis 6-endotoxin gene, it is recognized that
the corresponding hybridizable segments will hybridize
sense-strand to antisense-strand. That a segment may
be an antisense-strand does not detract from the
usefulness of such segments of genetic information
since cellular replication and transcription processes
will result in the formation of double-stranded DNA
containing both a sense and antisense-strain and since
the toxic proteins of this invention will generally be
expressed from the double-stranded DNA. Both sense and
antisense segments can likewise be used as
hybridization probes.

It is recognized that considerable modification of
DNA sequences by in vitro and in vivo means can take
place now that the gene sequence has been identified.
For example, one or more codons coding for a particular
amino acid can be replaced by other codons coding for
the same amino acid and in at least some cases by a
codon coding for a related amino acid (e.g., TTA, TTG,
GTT, GTG, GTA, and GTG all code for leucine while ATT
and ATC code for the similar amino acid isoleucine).
All such modifications in structure of the genetic
information that do not result in any change in the
structure or function of the protein coded for are
rightfully considered equivalents by those skilled in
the art of biochemistry.

By "cross-reactive" in this application is meant
that an antibody prepared against BTI 6-endotoxin
reacts with a toxic protein (which will be an antigen)
produced by the transformed strain. This protein
antigen need not be identical with the
6-endotoxin protein that provides the antigenic
determinant for the antigen used to produce the antibody. Naturally, if the protein antigens are identical, cross-reactivity will exist. However, it is also possible for the cross-reactive antigen to comprise at least in part a different amino acid sequence from that of the BTI δ-endotoxin used to generate the antibody.

A cell which has received genetic information coding for a toxic protein and which expresses the toxic protein, whether retained intracellularly or released into the culture medium, is referred to as a "producer cell." The phrase "genetic information" generally refers to DNA that codes for all or part of the toxic protein (and in preferred embodiments refers specifically to DNA that codes for δ-endotoxin). However, this phrase also encompasses RNA when used with an RNA virus or other vector based on RNA.

The term "vector" is well understood and is synonymous with the often-used phrase "cloning vehicle". A vector generally is non-chromosomal double-stranded DNA comprising an intact replicon such that the vector is replicated when placed within a unicellular organism, for example by a process of transformation. The vector is often a DNA plasmid or cosmid. However, the vector may also be derived from a phage and/or comprise RNA rather than DNA. Information originally present as RNA is converted by the cells' biochemical machinery into DNA, which then is expressed as the desired product.

"Host vector" means a vector into which heterologous genetic information can be inserted without destroying the ability of the vector to reproduce in a host microorganism. Use of the term "host" to describe both a vector and a microorganism does not imply that the vector is necessarily obtained from that microorganism.

Advances in biochemistry in recent years have led to the construction of "recombinant" vectors in which, for example, plasmids are made to contain exogenous DNA. In particular instances the recombinant vector can include heterogolous DNA, by which is meant DNA not normally present in the organism susceptible to transformation by the recombinant vector. The production of recombinant DNA vectors is well understood and need not be described in detail. However, a brief description of this process is included here for reference.

For example, a plasmid vector can be cleaved to provide linear DNA having ligatable termini. These termini are bound to exogenous DNA having complementary termini by ligation to provide a biologically functional recombinant DNA molecule having an intact replicon and a desired phenotypic property. A variety of techniques are available for DNA recombination in which adjoining ends of separate DNA fragments are tailored to facilitate ligation. Ligation refers to the formation of phosphodiester bonds between adjacent nucleotides, most often through the agency of the enzyme T4 DNA ligase. Thus, blunt ends may be directly ligated. Alternatively, fragments containing single strands at their adjoining ends that will bond to complementary single strands by hydrogen bonding can be ligated. Such single strands, referred to as cohesive termini, can be formed by the addition of nucleotides to blunt ends using terminal transferase. However, restriction endonucleases are most commonly used to produce cohesive termini. These enzymes cleave phosphodiester bonds in and around unique sequences of nucleotides of about 4-6 base pairs in length. Many restriction endonucleases and their recognition sites are known, the so-called EcoRI endonuclease being the most widely known. Typically, a plasmid or other cloning vehicle is cleaved, leaving termini each comprising a portion of the restriction endonuclease recognition site, and a cleavage product of exogenous DNA obtained with the same restriction endonuclease having complementary ends is inserted into the cleaved vector. Many variations of these techniques are known to those skilled in the art and can be used in the practice of the present invention.

The exogenous, i.e., donor, DNA used in producing a recombinant vector of the present invention is obtained from suitable cells of a donor identified as described above. DNA is generally obtained directly from prokaryotic cells (such as BTI) rather than as cDNA from messenger RNA, as is commonly done with eukaryotic cells. When fragments of DNA obtained from a BTI strain are inserted into a cloning vehicle, the resulting collection of DNA is referred to as a "donor gene library" since it contains DNA sequences coding for the gene products of the donor. Genes can then be selected from this library as was previously described.

It should be noted that the production of a gene library by itself is well known and that this discussion of producing a gene library is merely to point out the nature some of the known genetic manipulations that are carried out in the practice of the present invention.

The vector used in the production of the producer cell can be any vector that replicates in a unicellular organism. For example, many plasmids suitable for use in the practice of this invention have been described in recent U.S. patents such as U.S. Patents 4,273,875; 4,304,863; 4,419,450; 4,403,036; 4,363,877; 4,356,270; 4,349,629; and 4,332,901. Other vectors suitable for use in the practice of this invention include pAJ 655 (host bacteria, E. coli FERM-BP 136), pAJ 611 (host bacteria, E. coli FERM-BP 138), pAJ 440 (host bacteria, Bacillus subtilis ATCC 391391), pAJ 1844 (host bacteria, E. coli FERM-BP 137), and pAJ 3148 (host bacteria, Corynebacterium glutamicum ATCC 39137). Also preferred is the phage vector λgt11 which can be produced from publicly available phages according to the procedure described in Young et al, Proc. Natl. Acad. Sci. USA 80 (1983) 1194-1198. Briefly, this preferred phage vector can be produced as follows:

λgt-lac5 and λ540 (ΔB imm21 nin5) are cleaved with HindIII, and the fragments are pooled and then ligated with T4 DNA ligase. The desired phage recombinant produces turbid (imm21) blue (lac5) plaques when the DNA is transfected into E. coli BNN93 and cells are plated on medium containing the chromogenic indicator 5-bromo-4-chloro-3-indolyl β-D-galactoside (X-Gal). The λgt7-lac5-λ540 hybrid is then crossed with λgt4 (c1857 S100 nin5) and recombinants, grown at X-Gal plates. The presence of the amber mutation S100 is confirmed by examining relative plating efficiency on hosts that contain or lack the amber suppressor supF (BNN45 or BNN93, respectively). Finally, the lac5 c1857 S100 phage is mapped for Eco RI cleavage sites. The phage λgt11 contains a single Eco RI cleavage site.

Once a donor gene library or other source of a BTI

endotoxin gene is available, either according to the procedures described above or by any other procedure, recipient cells are transformed to produce a series of transformed cells containing the various donor DNA fragments. The incorporation of the recombinant DNA into recipient cells can be accomplished by treating the recipient unicellular microorganisms with calcium chloride to increase the permeability of the cell membranes to DNA, as is reported in Mandel et al, J. Mol. Biol., 53 (1970) 159. Other methods for transforming recipient cells include incorporating the recombinant DNA at a stage of growth when cells become capable of incorporating DNA (the so-called competent cell stage) as is reported in Young et al, Gene, 1, (1977) 153. Plasmids can also be incorporated into DNA recipients by forming protoplasts or spheroplasts of the DNA recipients (particularly used for yeast), as described in Chang et al, Mol. Gen. Genet., 168 (1979) 111. Any other method of transforming recipient cells can be used in the practice of this aspect of the invention.

The word "transformant" is used in this application in its broadest sense to refer to any recipient cell which has exogenous genetic information inserted therein. In addition to the more restricted meaning of transformant (a cell which has incorporated a plasmid), transformant in this application refers to cells which have exogenous DNA inserted into their chromosome (or chromosomes) as well as to cells which contain any recombinant vector as described above.

A microorganism is a progeny of a specific, identified strain when it is derived by cultivation of that strain. That a microorganism is a progeny of a specific strain need not be shown by proof of derivation from a deposit of the specific, identified strain since merely identifying a microorganism as, for example, B. megaterium VB131, does not limit that strain to a single deposited source. Using the techniques and publicly available starting materials identified in this specification, one skilled in the art of genetic engineering can readily make the same strains. Thus, a progeny of B. megaterium VB131 can be obtained from a deposit or from a separately produced but identical strain. Progeny of a named strain are therefore generally recognized by the identifying bichemical and physical characteristics of a microorganism, such as metabolic patterns, surface antigens, and ability to synthesize products, such as the toxic peptides of this invention, rather than by tracing the production history of the strain.

A "wild-type" stain of microorganism is one obtainable from a natural source and preferrably indicates a strain available from multiple environments rather than being adapted to a particular specialized environment. A wild-type strain has not been genetically engineered or artifically mutated.

The plural recipient cells which are transformed using a donor gene library are referred to as expression libraries since the transformants will express gene products from the recombinant DNA vectors that have been randomly inserted into the individual unicellular organisms. Typically, the transformants are cloned to produce a series of cell lines arising from individual original transformants. A sample can be taken from each cell line and (after lysing if desired) exposed to an antibody reactive with BTI endotoxin as described above. Binding interactions
between the antibody and the antigen expressed by the
transformant containing the proper gene can be detected
by any of the various methods available for this
process. For example, proteins from colonies of
transformants can be absorbed onto nitrocellulose
filter paper overlayed over the colonies followed by
addition of the antibody, washing to remove unreacted
antibody, and detecting protein-bound antibody with
radioactively labeled protein A. Cell colonies
identified in this manner can be grown in large
quantity to provide the desired amount of the gene
coding for the endotoxin antigen. At this point,
several variations are possible. The cells containing
the gene for the protein antigen can be allowed to
express the antigen and the antigen can be recovered
from the culture medium using known techniques.
Alternatively, the donor DNA fragment can be excised
from the first vector (e.g., by excision with the same
endonuclease used to cleave the plasmid and insert the
DNA originally), inserted into a new cloning vector,
and used to transform a recipient cell for large scale
production of the DNA or the endotoxin.

Host microorganisms (recipient cells) used in the
practice of biochemical aspects of this invention can
be any cell capable of protein production and able to
receive the genetic information discussed above.
Bacteria and yeast are preferred, particularly bacteria
and yeast that form spores during a part of their
normal life cycle, thereby allowing collection of the
spores for insecticidal use. Numerous examples of
suitable host microorganisms are given in the patents
listed in the following paragraph. However,
particularly preferred host microorganisms include

Bacillaceae bacteria capable of undergoing sporulation such as <u>Bacillus</u> and <u>Clostridium</u>, especially <u>Bacillus</u> <u>subtilis</u>, <u>Clostridium perfringens</u>, or a <u>Bacillus</u> <u>thuringiensis</u> strain other than <u>israelensis</u>, as well as <u>Bacillus megaterium</u>.

In addition to the above general procedures which can be used for preparing recombinant DNA molecules and transforming unicellular organisms in accordance with the practices of this invention, other known techniques and modification thereof can be used in carrying out the practice of the invention. In particular, techniques relating to genetic engineering have recently undergone explosive growth and development. Many recent U.S. patents disclose plasmids, genetically engineered microorganisms, and methods of conducting genetic engineering which can be used in the practice of the present invention. For example, U.S. Patent 4,273,875 discloses a plasmid and a process of isolating the same. U.S. Patent 4,304,863 discloses a process for producing bacteria by genetic engineering in which a hybrid plasmid is constructed and used to transform a bacterial host. U.S. Patent 4,419,450 discloses a plasmid useful as a cloning vehicle in recombinant DNA work. U.S. Patent 4,362,867 discloses recombinant cDNA construction methods and hybrid nucleotides produced thereby which are useful in cloning processes. U.S. Patent 4,403,036 discloses genetic reagents for generating plasmids containing multiple copies of DNA segments. U.S. Patent 4,363,877 discloses recombinant DNA transfer vectors. U.S. Patent 4,356,270 discloses a recombinant DNA cloning vehicle and is a particularly useful disclosure for those with limited experience in the area of genetic engineering since it defines many of the terms used in genetic engineering and the basic processes used therein. U.S. Patent 4,336,336 discloses a fused gene and a method of making the same. U.S. Patent 4,349,629 discloses plasmid vectors and the production and use thereof. U.S. Patent 4,332,901 discloses a cloning vector useful in recombinant DNA. Although some of these patents are directed to the production of a particular gene product that is not within the scope of the present invention, the procedures described therein can easily be modified to the practice of the invention described in this specification by those skilled in the art of genetic engineering.

All of these patents as well as all other patents and other publications cited in this disclosure are indicative of the level of skill of those skilled in the art to which this invention pertains and are all herein incorporated by reference.

The following microorganisms, which contain all of the genetic information necessary to carry out the present invention, are freely available from (among others) the indicated sources:

| Microorganism | Component | Public Source |
|---|---|---|
| BTI strain HD567(4Q4) | 112-kb BTI plasmid | Bacillus Genetic Stock Center |
| B. cerus GP7(6EI) | pBC16 Tc$^+$ plasmid | Bacillus Genetic Stock Center |

The Bacillus Genetic Stock Center, Dept. of Microbiology, Ohio State University, Columbus, Ohio [Telephone No. (614) 422-5550] will distribute cultures on request to those who can demonstrate their ability to safely handle the requested strains.

The following microorganisms have been deposited with the American Type Culture Collection, Rockville, Maryland, where they can be accessed under the stated identification numbers. These microorganisms also provide all of the necessary genetic information for practicing the invention in addition to the publicly available microorganisms listed above:

| Microorganism | Component | Deposit Number |
|---|---|---|
| BTI HD567-61-9 | 112-kb plasmid | 53025 |
| B. cereus GP7 (6EI) | pBC16 Tc$^+$ plasmid | 53027 |
| B. megaterium VT-1660 | host strain | 53026 |

A recombinant clone of Bacillus megaterium VB131 was constructed which contained a segment of the crystal toxin gene of Bacillus thuringiensis var. israelensis (BTI) in a vector plasmid pBC16. The B. megaterium clone strain harboring the chimeric plasmid produced irregular, parasporal, phase-refractile, crystalline inclusions during sporulation. The sporulated cells as well as the isolated crystal inclusions of the B. megaterium recombinant clones were highly toxic to the larvae of Aedes aegypti. One preferred aspect of the present invention is the DNA segment of the crystal toxin gene of Bacillus thuringiensis var. israelensis coding for an expressible toxic protein and described above in accordance with FIGURES 5 and 6 or having a nucleotide base sequence substantially as described in accordance with FIGURE 7 or 8. This segment, and equivalents thereto, can be incorporated into bacteria by using known techniques of DNA transfer and/or sub-cloning to make vectors or gene transfer vehicles and clones having the segment of the gene necessary to express a toxic protein. Preferably, this toxic protein has immunological cross-reactivity with an antisera for

δ-endotoxin  of Bacillus thuringiensis var.
israelensis and is toxic to insects, including those of
the order Diptera, especially larvae thereof.

Especially preferred nucleotide sequences for DNA
molecules and recombinant vectors of the invention
comprise all or part of the nucleotide sequence given
in FIGURE 7.  Of these, preferred segments are all
segments terminating in two of the restriction sites
listed in FIGURE 5 or 6 (e.g., SstI at 6 o'clock in
FIGURE 6 clockwise to ClaI at 7 o'clock; XbaI at 10
o'clock clockwise to ClaI at 11 o'clock; PvuII (3028)
to XbaI (6446) in FIGURE 5; and EcoRI (2918) to BamHI
(6771) in FIGURE 5).  All other segments obtainable
from cleavage at restriction sites identified in FIGURE
5 or 6 are specifically comtemplated to be preferred
embodiments of the invention, as if separately named at
this location.  Other preferred segments include all
segments that in which one end terminates at any of the
sites identifed as A, A', B, and B' in FIGURE 5,
whether such segments terminate at the other end at an
A, A', B, or B' site or at one of the identified
restriction sites (e.g., A to A', A to B, A' to B', A'
to B, A' to BamHI (6771), EcoRI (2918) to B', and StuI
(68) to B).  All other segments terminating at any A,
A', B, or B' site are likewise specifically
contemplated to be preferred embodiments of this
invention, as if separately named at this location.
Such preferred segments are still preferred when they
are part of a larger molecule (e.g., an EcoRI (2918) to
BamHI 96771) segment located within a larger DNA
molecule not equivalent to the sequence shown in FIGURE
7.).  Any peptide produced or producable by one of
these preferred nucleotide sequences is likewise a
preferred embodiment of this invention, as are any vectors or microorganisms containing these sequences.

The toxic proteins of the invention have been found to be toxic with respect to invertebrate pests, by which is meant insects of the class Insecta, especially of the order Diptera (especially larvae of mosquitoes and blackflies) and the like.

It is possible, once relatively large quantities of the endotoxin or the DNA which produces it are available, to determine the sequence of the molecular species as has been done in the present case (infra). Once this sequence is known, it is possible to synthesize the toxic protein using an automated peptide synthesizer or other suitable means of synthesis. Both laboratory techniques of synthesis, typically solid-phase synthesis, and automated peptide synthesizers are well known in the art. Since a given DNA sequence typically codes for a single peptide, the genetic information present in the cloned DNA segment can readily be used in a non-biological synthesis of a peptide. Artificial peptides produced using the genetic information preferably contain the entire coded region as described above. However, other artificial peptides having an amino acid sequence sufficiently identical to the sequence coded by the cloned DNA segment to result in a toxic peptide are also within the scope of the invention. An identical sequence is most preferred, but it will be readily recognized by those skilled in the art that modifications in the protein structure can be made without affecting the toxicity. For example, substitution of closely related amino acids (e.g., leucine for isoleucine) and production of peptides missing (or containing additional) terminal amino acids (preferably no more than 10 percent of the total amino acid content) are both possible. Modifications which fall within the scope of the present invention can readily be determined by their toxicity and cross-reactivity.

Once a recombinant DNA plasmid containing expressible heterologous DNA coding for a toxic protein having immunological cross-reactivity with δ-endotoxin of BTI has been prepared as described above and used to transform a bacterial host, or a toxic protein is produced in any other fashion, the endotoxin can readily be used to kill mosquitos and other dipteran aquatic insects. Although it is possible to release genetically engineered microorganisms carrying an endotoxin gene into the environment, a preferred method of use is the isolation of endotoxin crystals from a transformed bacterial strain and use of the isolated endotoxin as a larvicide. Although numerous methods for isolating crystal proteins from BT and BTI are known, a preferred technique involves the use of a density gradient and centrifugation. A preferred material for preparing density gradients is manufactured by E.R. Squibb & Sons, Inc., Princeton, New Jersey, and sold under the tradename "Renografin." Renografin is a dense, radiopaque dye normally used in veterinary diagnostics. Typically, cells expressing a toxic protein of the invertion or BTI δ-endotoxin are grown in culture to provide a mixture of cells and spore crystals, which is collected by scraping. The collected mixture is washed with water, sonicated to break up large clumps of particles, and layered (2 ml of mixture) onto a gradient made from 1 ml of 100% Renografin (bottom layer), 4.5 ml 80% Renografin (middle layer), and 4.5 ml 70% Renografin (top layer). Centrifugation at 13,000 r.p.m. in an SW41-2 rotor for two hours produces three bands of material (one at each interface). The middle band comprises substantially pure endotoxin crystals.

Alternatively, other techniques such as affinity chromatography using an antibody against normal BTI crystals can be used for endotoxin isolation. Particularly preferred is collection of spores from a sporulating bacterium that is expressing a toxic protein of the invention.

When isolated endotoxin is used in a natural environment, a preferable rate of application is from $0.2 \times 10^9$ to $10 \times 10^9$ I.U./acre, more preferably from $1 \times 10^9$ to $2 \times 10^9$ I.U./acre. An international unit (I.U.) is that rate of activity established as an international standard by the Institut Pasteur, Paris, France. Laboratory experiments have indicated that a concentration of 5 ng/ml of endotoxin produced from the 6.3-kb gene segment described herein produces 100% mortality in mosquito larvae at 36 hours. Any method of application which provides a similar concentration in the upper layer of aquatic surfaces to which a composition containing a similar BTI-like endotoxin of the invention is applied will produce similar results since aquatic larvae filter the crystal particles from the water as part of their natural feeding behavior.

Endotoxin of the invention may be applied in the same manner and at the same rate as natural BTI endotoxin, which is now being used commercially under the trademarks Teknar and Bactimos. Teknar is a water-miscible liquid containing 0.8% BTI endotoxin that is applied to water surfaces, typically by spraying, at a rate of 1-2 pints per acre. Bactimos is a 50% active wettable powder applied at the rate of 1 kg/hectare.

Many known insecticidal compositions comprising an active component and an inert carrier can likewise be adapted to the practice of the present invention by replacing the known active component with toxic protein of the invention. See, for example, U.S. Patents 4,325,937 and 4,328,203 and European Patent 59-707, which disclose such compositions.

For application, a compound of the invention ordinarily is applied most effectively by formulating it with a suitable inert carrier or surface-active agent, or both, and as active ingredient at least one toxic protein of the invention. The invention also provides a method of combating pests at a locus, which comprises applying to that locus a compound of the invention or a pesticidal composition according to the invention.

The term "carrier" as used herein means an inert solid or liquid material, which may be inorganic or organic and of synthetic or natural origin, with which the active compound is mixed or formulated to facilitate its application to the water surface, plant, seed, soil or other object to be treated. Any of the materials customarily employed in formulating pesticides (i.e., horticulturally acceptable adjuvants) are suitable. The active material can be a microorganism, a microorganism spore, a lyophilizate of a microorganism, or another preservation form of a microorganism as described herein or may be isolated toxic protein.

Suitable solid carriers are natural and synthetic clays and silicates, for example, natural silicas such as diatomaceous earths; magnesium silicates, for example, talcs; magnesium aluminum silicates, for example, attapulgites and vermiculites; aluminum silicates, for example, kaolinites, montmorillonites and micas; calcium carbonate; calcium sulfate; synthetic hydrated silicon oxides and synthetic calcium or aluminum silicates; elements such as, for example, carbon and sulfur; natural and synthetic resins such as, for example, coumarone resins and polyvinyl chloride and styrene polymers and copolymers; bitumen; waxes such as, for example, beeswax, paraffin wax, and chlorinated mineral waxes; solid fertilizers, for example, superphosphates; and ground, naturally-occurring, fibrous materials, such as ground corncobs.

Examples of suitable liquid carriers are water; alcohols such as isopropyl alcohol and glycols; ketones such as acetone, methyl ethyl ketone, methyl isobutyl ketone and cyclohexanone; ethers such as cellosolves; aromatic hydrocarbons such as benzene, toluene and xylene; petroleum fractions such as kerosene and light mineral oils; and chlorinated hydrocarbons such as carbon tetrachloride, perchloroethylene and trichloromethane. Also suitable are liquefied, normally vaporous and gaseous compounds. Mixtures of different liquids are often suitable.

The surface-active agent may be an emulsifying agent or a dispersing agent or a wetting agent; it may be nonionic or ionic. Any of the surface-active agents usually applied in formulating herbicides or insecticides may be used. Examples of suitable surface-active agents are the sodium and calcium salts of polyacrylic acids and lignin sulfonic acids; the condensation products of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohols or alkyl phenols, for example, p-octylphenol or p-octylcresol, with ethylene oxide and/or propylene oxide; sulfates or sulfonates of these condensation products; alkali or alkaline earth metal salts, preferably sodium salts, of sulfuric or sulfonic acid esters containing at least 10 carbon atoms in the molecule, for example, sodium lauryl sulfate, sodium secondary alkyl sulfates, sodium salts of sulfonated castor oil, and sodium alkylaryl sulfonates such as sodium dodecylbenzene sulfonate; and polymers of ethylene oxide and copolymers of ethylene oxide and propylene oxides.

The compositions of the invention may be prepared as wettable powders, dusts, granules, solutions, emulsifiable concentrates, emulsions, suspension concentrates and aerosols. Wettable powders are usually compounded to contain 25-75% by weight of active compound and usually contain, in addition to the solid carrier, 3-10% by weight of a dispersing agent, 2-15% of a surface-active agent and, where necessary, 0-10% by weight of stabilizer(s) and/or other additives such as penetrants or stickers. Dusts are usually formulated as a dust concentrate having a similar composition to that of a wettable powder but without a dispersant or surface-active agent, and are diluted in the field with further solid carrier to give a composition usually containing 0.5-10% by weight of the active compound.  Granules are usually prepared to have
a size between 10 and 100 BS mesh (1.676-0.152 mm), and
may be manufactured by agglomeration or impregnation
techniques.  Generally, granules will contain 0.5-25%
by weight of the active compound and 0-1% by weight of
additives such as stabilizers, slow release modifiers
and binding agents.  Emulsifiable concentrates usually
contain, in addition to the solvent and, when
necessary, cosolvent, 10-50% weight per volume of the
active compound, 2-20% weight per volume emulsifiers
and 0-20% weight per volume of appropriate additives
such as stabilizers, penetrants and corrosion
inhibitors.  Suspension concentrates are compounded so
as to obtain a stable, non-sedimenting, flowable
product and usually contain 10-75% weight of the active
compound, 0.5-5% weight of dispersing agents, 1-5% of
surface-active agent, 0.1-10% weight of suspending
agents, such as defoamers, corrosion inhibitors,
stabilizers, particularly for the protein, penetrants
and stickers, and as carrier, water or an organic
liquid in which the active compound is substantially
insoluble; certain organic solids or inorganic salts
may be dissolved in the carrier to assist in preventing
sedimentation or as antifreeze agents for water.

Of particular interest in current practice are the
water-dispersible granular formulations.  These are in
the form of dry, hard granules that are essentially
dust-free, and are resistant to attrition on handling,
thus minimizing the formation of dust.  On contact with
water, the granules readily disintegrate to form stable
suspensions of the particles of active material.  Such
formulations contain 90% or more by weight of finely
divided active material, 3-7% by weight of a blend of
surfactants, which act as wetting, dispersing, suspending and binding agents, and 1-3% by weight of a finely divided carrier, which acts as a resuspending agent.

Aqueous dispersions and emulsions, for example, compositions obtained by diluting a wettable powder or a concentrate according to the invention with water, also lie within the scope of the present invention. The said emulsions may be of the water-in-oil or of the oil-in-water type, and may have thick, mayonnaise-like consistency.

It is evident from the foregoing that this invention contemplates compositions containing as little as about 0.0001% by weight to as much as about 95% by weight of a compound of the invention as the active ingredient.

The compositions of the invention may also contain other ingredients, for example, other compounds possessing pesticidal, especially insecticidal, acaricidal or fungicidal properties, as are appropriate to the intended purpose.

The method of applying a compound of the invention to control pests comprises applying the compound, ordinarily in a composition of one of the aforementioned types, to a locus or area to be protected from the insects, such as the foliage and/or the fruit of plants or adjacent aqueous surfaces. The compound, of course, is applied in an amount sufficient to effect the desired action. This dosage is dependent upon many factors, including the carrier employed, the method and conditions of the application, whether the formulation is present at the locus in the form of an aerosol, or as a film, or as discrete particles, the thickness of film or size of particles, and the like. Proper consideration and resolution of these factors to provde the necessary dosage of the active compound at the locus to be protected are within the skill of those versed in the art. In general, however, the effective dosage of the compound of the invention at the locus to be protected (i.e., the dosage which the insect contacts) is of the order of 0.001 to 0.5% based on the total weight of the formulation, though under some circumstances the effective concentration will be as little as 0.0001% or as much as 2%, on the same basis.

The invention now being generally described, the same will be understood by reference to certain specific examples which are included herein for purposes of illustration only and are not intended to be limiting of the invention or any embodiment thereof, unless specified.

EXAMPLE:

Molecular cloning of the δ-endotoxin gene of Bacillus thuringiensis var. israelensis in Bacillus megatarium.

A transformant of Bacillus megaterium, VB131, was isolated which carries a 6.3 kb XbaI segment of the crystal toxin gene of Bacillus thuringiensis var. israelensis (BTI) cloned in a vector plasmid pBC16 to yield pVB131. The chimeric plasmid DNA from VB131 was introduced into a transformable Bacillus subtilis strain by competence transformation. Both the B. megaterium VB131 strain and the B. subtilis strain harboring the chimeric plasmid produced irregular, parasporal, phase-refractile, crystalline inclusions
(Cry$^+$) during sporulation. The sporulated cells as
well as the isolated crystal inclusions of the pVB131-
containing B. megaterium and B. subtilis strains were
highly toxic to the larvae of Aedes aegypti. Also, the
solubilized crystal protein preparation from
VB131[pVB131] showed clear immuno cross-reaction with
antiserum to the BTI crystal toxin. $^{32}$P-labeled pVB131
plasmid DNA showed specific hybridization with a 112-kb
plasmid DNA of Cry$^+$ strains of BTI, and no hybridization with other plasmid or chromosomal DNA of either
Cry$^+$ or Cry$^-$ variants.

Abbreviations used herein are defined as follows:
BT, Bacillus thuringiensis; BTI, Bacillus thuringiensis
var. israelensis; Cry$^+$, crystal-producing strain; Cry$^-$,
non-crystal producing strain; EtBr, ethidium bromide;
kb, kilobase pairs; PEG, polyethylene glycol; R,
resistance; SDS, sodium dodecyl sulfate; Tc,
tetracycline; [ ], indicates plasmid-carrier state.

MATERIALS AND METHODS

(a)  Strains.

A plasmid-free B. megaterium strain, VT1660, which
was derived from B. megaterium strain ATCC 19213 by
M.A. Von Tersch and B.C. Carlton [J. Bacteriol., 155
(1983), 872-877], was used as a host. B. subtilis
MI112, a highly transformable strain of B. subtilis,
was obtained from Dr. T. Tanaka, Osaka University,
Osaka, Japan. BTI strain HD 567-61-9, carrying only
the 112-kb plasmid of the usual complex array of the
prototype BTI strain HD567-1 [Gonzalez, J.M., Jr. and
Carlton, B.C.: "A Large Transmissible Plasmid is Required for Crystal Toxin Production in <u>Bacillus</u>
<u>thuringiensis</u> variety <u>israelensis</u>," <u>Plasmid</u> <u>11</u> (1984)
28-38], was used as the source of the 112-kb plasmid.
A 4.6-kb Tc$^R$ plasmid, pBC16, originally isolated from
<u>B. cereus</u> [Bernhard, K., Schrempf, H. and Goebel, W.:
"Bacteriocin and Antibiotic Resistance Plasmids in
<u>Bacillus cereus</u> and <u>Bacillus subtilis</u>," <u>J. Bacteriol.</u>
<u>133</u> (1978) 897-903], was originally transformed into
<u>B. megaterium</u> VT1660 by M.A. Von Tersch (strain VT
1650) and was used as the source of the vector pBC16
plasmid in this experiment.

(b)  DNA isolation.

In some experiments, the 112-kb plasmid DNA from
<u>BTI</u> HD567-61-9 was a generous gift from J.M. Gonzalez,
Jr.  In other experiments this 112-kb plasmid and the
plasmid vector DNA, pBC16, from <u>B. megaterium</u> VT1650
were isolated according to the method of Gonzalez,
J.M., Jr. and Carlton, B.C.:  "Patterns of Plasmid DNA
in Crystalliferous Strains of <u>Bacillus thuringiensis</u>,"
<u>Plasmid</u> <u>3</u> (1980) 92-98.  The plasmid DNAs were further
purified by two passages on CsCl-EtBr buoyant density
gradients.  The EtBr was extracted and the DNA's were
precipitated as described by Davis, R.W., Botstein, D.
and Roth, J.R. (1980) in "Advanced Bacterial Genetics
(A Manual for Genetic Engineering)", pp 126-127 (Cold
Spring Harbor Laboratory, NY).

(c)  Restriction and ligation.

The protocols used for restriction endonuclease
digestion and ligation were essentially as described by
Maniatis, T., Fritsch, E.F. and Sambrook, J.:  "Enzymes
Used in Molecular Cloning," in <u>Molecular Cloning</u>:

A Laboratory Manual, Cold Spring Harbor Laboratory, New York (1982) pp. 97-148. 5.5 µg of 112-kb BTI plasmid DNA and 0.10 µg of pBC16 vector DNA were separately digested in 20 µl each of 1x high salt restriction buffer with 6 units each of the restriction enzyme XbaI (Bethesda Research Labs (BRL), 10 units/µl)). The 112-kb plasmid DNA was digested at 37°C for 30 min, and the pBC16 plasmid DNA was digested at 37°C for 60 min. The two plasmid DNA digests were mixed and precipitated with absolute ethanol in the presence of 1.0 M (final concentration) Na acetate, and the pellet was dissolved in 30 µl of 1X ligation buffer [Alton, N.K.: "A Molecular Analysis of the Expression of the Neurospora crassa Catabolic Dehydroquinase Gene in Neurospora crassa and Escherichia coli," Ph.D. Dissertation (1981), Univeristy of Georgia, Athens, GA (U.S.A.)] containing 5 units of T4 DNA ligase (BRL, 10 units/µl). Ligation of the fragments was carried out at 16°C for 20 h. The ligated DNA mixture was used directly in the transformation experiments.

(d) Protoplast transformation of B. megaterium VT1660.

PEG-induced protoplast transformation of B. megaterium VT1660 was carried out as described by Von Tersch, M.A. and Carlton, B.C.: "Megacinogenic Plasmids of Bacillus megaterium," J. Bacteriol. 155 (1983) 872-877. After exposure to the ligated DNA mixture, the protoplasts were allowed to incubate at 30°C for 2 h and were plated on supplemented regeneration plates containing 20 µg/ml Tc. The plates were incubated at 30°C for 48 h before scoring $Tc^R$ transformants. The $Tc^R$ transformants thus obtained were allowed to sporulate at 30°C and were stored at 5°C on nutrient agar (Difco) plates supplemented with

1 mM mgSO$_4$, 0.12 mM MnCl$_2$, 0.2 mM CaCl$_2$, 0.1% glucose, and 20 µg/ml Tc [Tobey, J.F. and Yousten, A.A.: "Factors Affecting Production of Amylase by Bacillus thuringiensis," in Developments in Industrial Microbiology, Vol. 18, American Inst. Biol. Sci., Washington, D.C. (1977), pp. 499-510].

(e)  Screening of recombinant clones.

The Tc$^R$ transformants of B. megaterium VT1660 were analyzed on 0.8% agarose gels by the slot-lysis electrophorectic procedure of Von Tersch and Carlton (1983).  Transformants containing plasmid(s) larger than the pBC16 vector were identified by the slower mobilities of their plasmids on these gels, and these strains were assayed for their ability to produce δ-endotoxin by mosquito bioassay.

(f)  Bioassay on mosquitos.

Suspensions (0.3 ml) of logarithmically growing cells (in 0.8% nutrient broth (Difco)) of the strains containing recombinant plasmids were plated in duplicate on supplemented nutrient agar plates (5.5 cm diameter) containing 20 µg Tc/ml.  Control plates of BTI strain HD567-61-9 and B. megaterium VT1650 were also prepared.  The plates were incubated at 30°C for several days until the cultures were well sporulated (>80% phase refractile spores).  To each plate, 5 ml deionized water and 15 second-instar larvae of Aedes aegypti, previously starved overnight, were added.  The plates were incubated at 27°C for 24 h.  The numbers of dead larvae in each of the plates was scored at 0.5 h, 2 h, 8 h, and 24 h.  One strain, VB131, was kept for further testing.

The parasporal inclusions produced by strain
VB131[pVB131] and the crystals from the reference BTI
HD567-61-9 strain were isolated on Renografin (E.R.
Squibb & Sons, Inc., Princeton, NJ) by the method of
Gonzalez, J.M., Jr., Brown, B.J. and Carlton, B.C.:
"Transfer of Bacillus thuringiensis plasmids Coding
for δ-Endotoxin," Proc. Natl. Acad. Sci. USA 79 (1982)
6951-6955. Crystal inclusions were solubilized among
strains of B. thuringiensis and B. cereus at room
temperature in 0.1 M $HCO_3$ buffer, pH 10.2, containing
10mM dithiothreitol for 12 h (Huber et al., 1981), and
the protein concentrations were determined according to
Lowry, O.H., Rosebrough, N.J., Farr, A.L. and Randall,
R.J.: "Protein Measurement with the Folin Phenol
Reagent," J. Biol. Chem. 193 (1951) 265-275. Measured
amounts of whole crystal protein preparations and 20
starved second-instar larvae of Aedes aegypti were
added to 20 ml deionized water in 25-ml plastic cups.
This assay was performed in triplicate at 27°C, and the
numbers of dead larvae in each cup were scored at 24
and 36 h.

(g)   Immunological characterization of crystal protein.

The immunological cross-reactivities of crystal
proteins from BTI HD567-61-9 and VB131 strains were
compared on double immunodiffusion plates using rabbit
antiserum to the BTI crystal protein.

(h)   Nick-translation of cloned plasmid DNA and
      Southern blot hybridization.

pVB131 plasmid DNA from the strain that showed
mosquito toxicity was isolated and purified twice on
CsCl-EtBr gradients. The DNA was labeled with
$[\alpha-^{32}P]$dATP (Amersham, sp. act. 700 Ci/mmol) by nick-translation as described by Maniatis et al. (1982). By this protocol ~10% incorporation of the $^{32}$P-label into the plasmid DNA was obtained in 4 h. DNA-DNA hybridization blot experiments [Southern, E.M.: Detection of Specific Sequences Among DNA Fragments Separated by Gel Electrophoresis," J. Mol. Biol. 98 (1975) 503-517] using the $^{32}$P-labeled pVB131 DNA as a probe were performed exactly as outlined by Maniatis et al. (1982).

(i) Introduction of pVB131 plasmid DNA into B. subtilis and the biological activity of B. subtilis transformants.

B. subtilis strain MI112 was transformed with 5 µg of purified pVB131 plasmid DNA by the competence DNA transformation protocol of Anagnostopoulos, C. and Spizizen, J.: "Requirements for Transformation in Bacillus subtilis." J. Bacteriol. 81 (1961) 741-746. A B. subtilis transformant, SB131, produced irregular crystal inclusions during sporulation (Fig. 1). The sporulated cells and isolated crystal inclusions of SB131 were tested for their biological activity on mosquito larvae as described previously.

(j) Restriction endonuclease mapping of the XbaI fragment containing the cloned crystal toxin gene.

The relative locations of several restriction sites were determined using standard multiple digestion methods. Phage λ DNA digested with HindIII and pBR322 DNA digested with HaeII served as $M_r$ standards.

(k) DNA sequencing.

The fragment from XbaI restriction endonuclease incubation was subjected to the standardized dideoxy method of W. Gaastra and B. Dudega, "Techniques in Molecular Biology", Ed. J.M. Walker and W. Gaastra, pp 291-7, MacMillan Publishing Co., N.Y. (1983), and references therein, to determine the DNA nucleotide base sequence set forth in FIGURE 7.

RESULTS

(a)  Transformation of B. megaterium VT1660 and selection of recombinant clones.

Purified 112-kb plasmid DNA isolated from strain HD567-61-9 was digested with XbaI and mixed with similarly-digested pBC16 plasmid DNA propagated in B. megaterium VT1650.  Following ligation with T4 DNA ligase the mixture was added to protoplasts of plasmid-less B. megaterium strain 1660.  Of the 476 Tc$^R$ transformants recovered 28 contained recombinant plasmids with inserts ranging from ~0.8-~15.0 kb. Examination of these strains by phase-contrast microscopy (Fig. 1) revealed one isolate, VB131, which produced phase-refractile irregular parasporal inclusions similar to those observed in Cry$^+$ strains of BTI.

(b)  Mosquito bioassays.

Of the 28 strains containing recombinant plasmids only VB131 elicited mosquito toxicity.  While plates containing sporulated VB131 showed 100% killing of mosquito larvae in ~6 h, plates containing BTI HD567-61-9 killed 100% of the larvae in ~1 h at 27°C (Table I).  When the isolated inclusions from VB131 were tested for larvicidal activity in a standard cup assay, 25 ng/ml of the unsolubilized crystal protein produced 100% mortality in ~36 h, whereas 5 ng/ml of unsolubilized crystal protein from BTI HD567-61-9 showed 100% mortality in ~36 h (Table II). The different activity clearly shows that the endotoxin, while retaining toxic activity, was not identical to the natural product. The solubilized crystal protein preparations of both VB131 and HD567-61-9 strains showed ~50-60-fold lower activity than the unsolubilized crystal preparations (data not shown).

Table I. Plate assay for larvicidal activities of sporulated cultures of various Bacillus strains and clones

| Strain | Number of larvae/plate | Dead larvae/plate | | | |
|---|---|---|---|---|---|
| | | 0.5h | 2h | 8h | 24h |
| BTI HD567-1 | 15 | 15 | 15 | 15 | 15 |
| BTI HD567-61-9 | 15 | 15 | 15 | 15 | 15 |
| B. megaterium VT1650 | 15 | 0 | 0 | 0 | 1 |
| B. megaterium VB131 | 15 | 0 | 2 | 15 | 15 |
| B. subtilis SB131 | 15 | 0 | 2 | 14 | 15 |

Table II. Cup assay for larvicidal activities of crystal protein preparations.

| Strain | Crystal protein concentration in bioassay cup (ng/ml) | larvae/cup | Dead larvae/cup | |
|---|---|---|---|---|
| | | | 24h | 36h |
| BTI HD567-61-9 | 5 | 20 | 15 | 20 |
| B. megaterium VB131 | 25 | 20 | 10 | 20 |
| B. subtilis SB31 | 25 | 20 | 10 | 20 |

The larvicidal activities of the sporulated cells and purified crystal inclusions of the B. subtilis SB131 transformant were very similar to those of VB131 (Tables I and II).

(c) Detection of crystal antigen in B. megaterium VB131.

The solubilized crystal proteins from both HD567-61-9 and VB131 strains showed clear cross-reactions against the rabbit antiserum to the BTI crystal protein when tested by the double immunodiffusion method (Fig. 2).

(d) Southern blot hybridization.

Nick-translated pVB131 DNA was as a probe to determine the location(s) of δ-endotoxin-coding gene sequence(s) in various plasmid DNAs of several Cry[+] and Cry⁻ variants of BTI that were separated on agarose gels [Gonzalez et al. (1982)] by the method of Southern (1975). Among the "normal" Cry⁺ variants, the probe specifically hybridized with the 112-kb plasmid (Fig. 3B, lanes a, e and g). Two Cry⁺ variants of BTI, HD567-39 and HD567-40 (lanes b and c), that did not contain the 112-kb plasmids, respectively, also hybridized with the pVB131 probe. These two plasmids have been postulated to be derived from the 112-kb plasmid of BTI, most likely by integration of the native plasmids into, and subsequent excision from, the chromosome [Gonzalez and Carlton (1984)]. Two Cry⁻ variants, HD567-42 (lane d) and HD567-61 (lane f), did not show any hybridization with the probe. DNA from the plasmid-free B. megaterium strain VT1660 (lane l) showed no sequence homology to pVB131 DNA. B. subtilis MI112 carrying the vector pBC16 plasmid DNA (lane n) and the recombinant plasmid DNAs from B. megaterium VB31 (lane j) and B. subtilis SB131 (lane m) showed strong homology to the probe.

XbaI digestion of pVB131 plasmid DNA yielded the linearized 4.6-kb vector DNA and three additional fragments (Fig. 4A, lane c). From the sizes of these three fragments (one large fragment of 2.7 kb and two poorly-resolved fragments of about 1.8 kb each), the size of the insert was determined to be 6.3 kb. Three fragments of identical size were seen among the 23 to 24 restriction fragments of XbaI-digested 112-kb DNA (Fig. 4A, lane b). These three fragments, plus two other larger fragments which appear to be incomplete digestion products, showed specific hybridization with the pVB131 probe (Fig. 4B, lane b). In attempts to subclone the toxin gene, B. megaterium strains containing either the large XbaI fragment or one of the two small fragments of pVB131 were generated (data not shown). None of these subclones produced crystal inclusions or significant larvicidal activity.

(e)   Restriction endonuclease mapping of the XbaI
      fragment containing the cloned crystal toxin gene.
      The 6.3 kb XbaI fragment derived from pVB31 was
shown to contain two restriction sites for each of the following enzymes: XbaI, ClaI and PvuII. Unique restriction sites were found for StuI, SstI, Eco RI, PstI and HindIII (Fig. 5).

DISCUSSION

Twenty-eight recombinant clones were obtained when the toxin-coding 112-kb plasmid DNA of BTI strain HD567-61-9 was restricted with XbaI, and the fragments ligated into the unique XbaI site of the plasmid vector pBC16 and transformed into a B. megaterium host. Sporangia of one transformant, VB131, carrying the hybrid plasmid pVB131, were found to produce irregular parasporal inclusions similar to those of the reference BTI strain HD567-61-9. Of the 28 isolates tested for their ability to produce δ-endotoxin toxic to larvae of Aedes aegypti, only VB131 was active. The crystal inclusions from VB131 also showed immunological similarity to those of HD567-61-9. While the whole crystal proteins from both HD567-61-9 and VB31 ultimately produced 100% mortality to mosquito larvae, the BTI crystal inclusions acted about 5 times faster at equivalent protein concentrations.

The invention now being fully described, it will be apparent to one of ordinary skill in the art that many changes and modifications can be made thereto without departing from the spirit or scope of the
invention as set forth herein.

## Claims

1. A recombinant vector capable of replicating in a host microorganism, wherein said recombinant vector comprises a host vector having inserted therein expressible heterologous genetic information coding for a toxic protein having a toxic property of δ-endotoxin from <u>Bacillus</u> <u>thuringiensis</u> var. <u>israelensis</u>.

2. The recombinant vector of Claim 1, wherein said heterologous genetic information comprises a segment hybridizable with a <u>Bacillus</u> <u>thuringiensis</u> var. <u>israelensis</u> δ-endotoxin gene.

3. The recombinant vector of Claim 1, wherein said protein is less toxic toward mammalian cells than δ-endotoxin from a wild-type strain of <u>Bacillus</u> <u>thuringiensis</u> var. <u>israelensis</u>.

4. The recombinant vector of Claim 3, wherein said wild-type strain is HD567.

5. The recombinant vector of Claim 1, wherein said toxic protein is immunologically cross reactive with δ-endotoxin from <u>Bacillus</u> <u>thuringiensis</u> var. <u>israelensis</u>.

6. The recombinant vector of Claim 1, wherein said segment contains a nucleotide sequence identical to a nucleotide sequence of a δ-endotoxin gene present on a 112-kb plasmid of <u>Bacillus</u> <u>thuringiensis</u> var. <u>israelensis</u> bacterium.

7. The recombinant vector of Claim 6, wherein said bacterium is a progeny of strain HD567-61-9 or a mutant thereof.

8. The recombinant vector of Claim 1, wherein said recombinant vector is a DNA plasmid containing heterologous genetic information comprising a 6.3-kb segment between two XbaI restriction sites.

9. The recombinant vector of Claim 8, wherein said 6.3-kb segment contains two internal XbaI restriction sites wherein XbaI restriction endonuclease divides said 6.3-kb segment into a 2.7-kb segment and two 1.8-kb segments.

10. The recombinant vector of Claim 8, wherein said 6.3-kb segment contains a sequential series of restriction endonuclease sites comprising XbaI, StuI, SstI, ClaI, XbaI, Eco RI, PvuII, PstI, XbaI, PvuII, ClaI, HindIII, and XbaI.

11. The recombinant vector of Claim 1, wherein said host vector is pBC16.

12. An isolated DNA or RNA molecule, wherein said molecule codes for a toxic protein having a toxic property of δ-endotoxin from Bacillus thuringiensis var. israelensis.

13. The molecule of Claim 12, wherein said molecule is DNA.

14. The molecule of Claim 13, wherein said molecule contains a 6.3-kb segment between two XbaI restriction sites.

15. The molecule of Claim 14, wherein said 6.3 kb segment contains a sequential series of restriction endonuclease sites comprising XbaI, StuI, SstI, ClaI, XbaI, Eco RI, PvuII, PstI, XbaI, PvuII, ClaI, HindIII, and XbaI.

16. The molecule of Claim 12, wherein said molecule comprises a nucleotide sequence of about 3531 bases, said sequence substantially as set forth in Figure 7, base pair position number 2916 to 6446.

17. The molecule of Claim 12, wherein said molecule comprises a nucleotide sequence of about 3579 bases, said sequence substantially as set forth in Figure 7, base pair position number 2916 to 6485.

18. A microorganism containing a recombinant vector which comprises expressible heterologous genetic information capable of expressing in said microorganism a toxic protein having a toxic property of δ-endotoxin from Bacillus thuringiensis var. israelensis.

19. The microorganism of Claim 18, wherein said microorganism comprises a host bacterial strain containing a recombinant plasmid and said plasmid comprises a vector capable of reproducing in said host and heterologous DNA coding for said protein.

20. The microorganism of Claim 19, wherein said host bacterial strain is a progeny of Bacillus megaterium VT-1660 or a mutant thereof.

21. The microorganism of Claim 19, wherein said microorganism is a progeny of Bacillus megaterium VB131 or a mutant thereof.

22. A microorganism spore, wherein said spore is produced by a microorganism of Claim 18.

23. A method of killing insects susceptible to δ-endotoxin of <u>Bacillus thuringiensis</u> var. <u>israelensis</u>, which comprises:

applying an insecticidally effective amount of a toxic protein to a locus containing said insect, wherein said protein is expressible by a microorganism containing a recombinant vector of Claim 1 containing heterologous DNA coding for said protein and said protein is not identical to δ-endotoxin from a wild strain of <u>Bacillus</u> <u>thuringiensis</u> var. <u>israelensis</u>.

24. An insecticidal composition comprising a microorganism, a microorganism spore, a lyophilizate of a microorganism, or other preservation form of a microorganism containing a recombinant vector according to Claim 1.

25. A composition according to Claim 28, which comprises spores and upon dilution produces a culture broth suitable for germination of said spores.

26. A toxic protein having an amino portion substantially identical to the amino portion of δ-endotoxin from <u>Bacillus thuringiensis</u> var. <u>israelensis</u> and a carboxy-terminus portion different from the carboxy portion of said δ-endotoxin, wherein said toxic protein exhibits a toxic property of said δ-endotoxin.

27. The toxic protein of Claim 26, wherein the amino-terminus portion of said toxic protein is substantially identical to the amino acid sequence of δ-endotoxin  as shown in Figure 8.

28. The toxic protein of Claim 26, wherein the amino-terminus portion of said toxic protein is identical to the amino acid sequence of  δ-endotoxin shown in the Figure 8.

29. An insecticidal composition comprising a toxic protein according to Claim 26 in a form suitable for insecticidal use, optionally including a diluent, carrier or excipient.

30. A method for identifying a gene capable of expressing a toxic protein, which comprises:

hybridizing (1) a probe consisting essentially of labeled DNA or RNA complementary to a δ-endotoxin gene segment with (2) a δ-endotoxin gene source;

selecting DNA segments from said gene source that hybridize with said probe;

forming a recombinant DNA vector from said DNA segments and a vector,

transforming a host microorganism with said recombinant DNA vector, and

selecting transformed microorganisms that express a protein toxic to an organism susceptible to δ-endotoxin .

DR. ING. ... ...D GIAMBROCONO
DR. MARZI ... ENGH
DR. ING. LU S FRIGNOLI
DR. ING. GIORGIO LUKSCH
DR. LUISA MAGNOLI
SIG. GIOVANNI VUCETICH
DR. ONOFRIO GOFFREDO
SIG. MA. TRI OVSCH
SG. FABIO GIAMBROCONO
DR. LUISA GARHASSIN
DR. LUCIANA LONZA

UFFICIO DI BERGAMO
DR. ING. ANGELO ZAPPELLA

UFFICIO DI REGGIO EMILIA
DR. ING. ALFONSO COLLI
CONSULENTE CAV. UFF. SCANIO LARI

CONSULENTI LEGALI
AVV. ERMINIO PARINI
AVV. ALESSANDRA MUÑOZ
DR. PROC. MARCO ANDREOLINI

VIA ROSOLINO PILO, 19/
20129 MILANO
TEL. (02) 2041251
GIAMBROCONO
TELEX 321337 ISTPA1
TELEFAX (02) 204 32 9

**0195285**

For the purpose of publication, correction(s)

☒ allowed

☐ allowed with exception of the deleted points

☐ not allowed

Signature: _____ date: _____

Receiving Section

EUROPEAN PATENT OFFICE
P.B. 5818 Patentlaan, 2
2280 HV RIJSWIJK (ZH)
NETHERLANDS

Ns. Rif. M 3971 NG/pl   Vs. Rif.

Milano  May 6, 1986

Re: European Patent Application No. 85102547.6
in the name of UNIVERSITY OF GEORGIA RESEARCH
FOUNDATION, INC.
=================================================

Referring to the mentioned european patent application, we
are herewith enclosing the EPO Form 1037 with the formal
drawing in n. 16 tables in triple copy.

Furthemore, please note that on fig. 8-1 and 8-2 of the pro
visional drawings, some segments have been inadvertently
omitted that on the contrary appear on the final drawings.

Please acknowledge safe receipt of this letter by returning
the enclosed copy.

With best regards,

Bauer J. A.

B.M. HARTMANN  28 MAY 1986

Very truly yours,

Dr.Ing. A. Giambrocono

Encl.

Cap. Soc. 50.000.000 - CCIA 872506 - Trib. Mi 200947 - Cod. Fiscale 01698140157

1/16

FIG. IB

FIG.ID

FIG. IA

FIG.IC

21/16

FIG.2

FIG. 3A

a b c d e f g h i j k l m n

FIG. 3B

a b c d e f g h i j k l m n

41/96

# FIG. 4A

# FIG. 4B

M.W.(kb)

23.7

9.46

6.66

4.26

2.26
1.96

FIG. 5

5/16

Xbal (0)  A (0)
StuI (68)
  1.10
SstI (1256)
  0.95
ClaI (2255)
  0.56
XbaI (2756)
Eco RI (2840,2918)  A' (2916)
PvuII (3028)
PstI (3105)
  1.54
XbaI (4598)
  0.70
PvuII (5337)
  0.44
ClaI (5826)
  0.50
HindIII (6345)
XbaI (6446)  B (6446)
BamHI (6771)  B' (6485)

2.7
1.8
1.8

FIG. 6

7/16

1-050 TCTAGAAAAA ATAATGTTAT TTGTACTTGG TGGTATGTAA GTTTCTTAAG

51-100 TTGATGGGTA TGGTGAGGCC TTATAAAAAA ATAGCGTTAA CGAGCATGTA

101-150 TCGTGACCTC AAGTAGTAAA AAAGGCAAGC CTCTACATGA GTATTTTGGG

151-200 TTCTGTTGCA AAGTTTTTGG AGTGAAGTTA AAATATAGAG AAAATGGGGC

201-250 GAGGAGAAGG ATGCATGGGA TATTTTAAAG GGAAACAATT CAAAAAAGAT

251-300 ATTATTTTGG TAGCCGTTGC GTATTACTGT CGTTTTTCTT TAAGTTATCG

301-350 TGATGTGTCT GAGATCCTAA AAGAACAAGG AGTTTCTGTA CATCCCACAA

351-400 CCATCATGCG TTGGGTTCAT GAATATGGCA ATTTGATTTA TCAAATATGG

401-450 AAGAAGAAAA CCAGTCTGCA CATCATGTTT GGCATGTAGA TGAGACGTAC

451-500 ATCAAAGTGA AAGGGGAGTG GTCTTATCTG TATCGTGCGA TTGATAGTAA

501-550 TGGATATACA CTTGATTTTC AACTTCGAAA AACACGAGAT CATCAAGCAG

551-600 CATATGCCTT TATAAAAAGG TTAGCAAAGC AATTTGGAAC CGAACGGTTC

601-650 TTACAACAGA TCAAGCTCCT GCACTGCTTT GTGCGTTCAA AAAACTGAAA

651-700 AATAATGGCT TTTATGTGCA TATAACACAT TGTACGGTTA AGCACCTTAA

701-750 TAACCTGATT GAACAAGATC ATCGACATGT CAAACGACGT TTTGCCAAAT

751-800 CTTCTGGATT CCAAAATATC CGTCATGCTT CACGTACATT GAAAGGAATC

801-850 GAAACGATTC ATGCGATATA CAAACAAAAG AGAAGTCAAA TTCCAGACTT

851-900 CTCTTTTTCT ACGTACAAGG AATTACAGGA GTTATTCAGA ACCGCTTAAA

901-950 CTATAACTAT ATTTTGTAAA CAAGTCTGTG TTTTTTCAAA CTTTGCAACA

951-000 CACCTCAAAA AGCTATCTGT TTATCCCATC AGTATGTGAA CCGTTTGTTT

1001-050 CACTTGTTTA TCGTCCACTC GATAAAATAA CTTCTGTTCC TTTGCGTTCA

1051-100 TGTGATAACA CTTTATGATT TCTCATTTTA TGAAGGTGTT GTGAAACCGT

1101-150 AGATTGTGGT AATTTTAAAA ATTTTTTGTA ATTCGGTAAC AGTTTAAAGC

1151-200 ATTTTTCTCT AATAATTGAT GTACAATTTG TAGACGGAAA GGATGTGCCA

1201-250 ATACCCTCAG TATTTCCGCA CTTCGCTCGA GTTCATAATT AAGATCGTTG

1251-300 AGCTCCATAT AAAAAACATT TGTCATCTTC TTTCGCCTCA TTCTTTTTTT

1301-350 AGGGTATTTT CTCATATACA CGTTTTTTCG TTAGCAAAGA ATCTAACATG

# FIG.7-1

8/16

1351-400 TTACACTTAC AAACTATCTT GCACCGTTCT CTTTCTCCCT TTATACTGAA

1401-450 ACAAGTATAT ATCACAATAA GAGGTGACTC ATGAACAAAT CAGAATTAAT

1451-500 CAAACAAGTC GCAATACAAT CTGAACTAAA AAAGCCACAA GCAAGTCTTG

1501-550 CAGTAGATGC CGTATTAGAG TCCATTCAAC ATGCACTACA AAATGGAGAC

1551-600 CATGTACAGC TATTTGGTTT CGGTTCATTT GAAGTAAAAG AACGTGCTGC

1601-650 ACGTGAAAGG ACGCAATCCG CATACTGGTG AGGCACTTAC TATTCCTGCG

1651-700 GGAAAACAC CTGCCTTTAA AGCAGAAAAG CATTAAAAGA AGCTGTAAAC

1701-750 GCTAAATAAT ATTTCTTTAA CAGTAGCATC AATCCAGTAG GAATCATCGT

1751-800 GCATTTTATT TTAGAATCGA ATCGTCGAGA TTGCATGTTT ATATACAGCT

1801-850 GTTGCTTACC TTCAACTTCT ATTAAAACAG AATAAGTATC AAATCCTCTT

1851-900 ATTATTCCTT TTACATGCAT CCTTTTAACA GATTAAAGTG ATATCCTTCC

1901-950 TCTTTTGAAC GCCTCTTGCA CATTGGTTCC TGCAAAGATA GCATGTTCTT

1951-000 GCTTTTATCC TTTTGTGCTT GTAATTTCAC TTCCACGAGT TCGCCCCCTA

2001-050 TTCTTTTCAT ACTGTTACTT TGTATTGTTC TAATTCTTCC ATAAGTTTTG

2051-100 TCCGTCTTCT GGGCTCAACG TCAGCTTTCC ATCCGCATGT TTGATATTCT

2101-150 GGGAAGAAAT CTCCCCTGTG ATGGGACAAG TACCATACGA TTGATATTTC

2151-200 TGATGAATAT TCGTTTCGCC TTCATCAAAT ATTTCTAATG GTTCTTTTTC

2201-250 GTGAATATTC AGTGTACGCC TCGTTTCCCT TGGTATCACA ATTCGACCAG

2251-300 CTCATCGATT TTTCGTACAA TACCGGTTGC TTTCATAGAA ATGCCCTCCT

2301-350 TTTGTACATA TAAAATATCT CACATCTTAT GTGACCGTTT TCATTTTTTA

2351-400 AAAACTGCAT ATACTATAAA AACGTTGATA TCAATCTGTA GAAATAGCGA

2401-450 GGTTTCCCAT GTCTACATCT GATGTTACCC ACTTACAAAA ACAAATTGAA

2451-500 GAAAAAAGA AACAACTGCG ACAGTTGGTA AGAGCATACG GATATACAGA

2501-550 CCCCCTAATT TTTGCCTGTA GCCAAGAATT AGATCAACTC GTGTATCGTT

2551-600 TTATGCGTGA TTATTCTCCA CAAAAAGTTA CATGTAAAAA ACGATTCCAA

2601-650 TTCATCAATA GAATTGGAAT CTCACTATAC ATATGTCCTC TATATTTTAC

2651-700 CATAACTGGA GTCTATGTGG ACTAAATAAA CCAACTTCTG TATAAAAACA

2701-750 TGCCTCTTGG TATATATTAG GGATAACAAT TCCACCCTAC AACATTGATT

2751-800 GTTACTCTAG ATAAGAATTG TTCATAGGAA TCCGTATCAA TTTTTTTCAAG

2801-850 GAATATGTAT TTGCACTTTT GGTCTTTTTT AAATCGTATG AATTCAAAAT

2851-900 AGTTTATATC AATCTTGTTA CACCAGAAAA AGATTGTATC CAATGTGAAT

# FIG. 7-2

2901-950 ATGGGAGGAA TAAATATGAA TTCAGGCTAT CCGTTAGCGA ATGACTTACA

2951-000 AGGGTCAATG AAAAACACGA ACTATAAAGA TTGGCTAGCC ATGTGTGAAA

3001-050 ATAACCAACA GTATGGCGTT AATCCAGCTG CGATTAATTC TTCTTCAGTT

3051-100 AGTACCGCTT TAAAAGATGC TGGAGCTATC CTTAAATTTG TAAACCCACC

3101-150 TGCAGGATCT GTCTTAACCG TACTTAGCGC GGTGCTTCCT ATTCTTTGGC

3151-200 CGACTAATAC TCCAACGCCT GAAAGAGTTT GGAATGATTT CATGACCAAT

3201-250 ACAGGGAATC TTATTGATCA AACTGTAACA GCTTATGTAC GAACAGATGC

3251-300 AAATGCAAAA ATGACGGTTG TGAAAGATTA TTTAGATCAA TATACAACTA

3301-350 AATTTAACAG TTGGAAAAGA GAGCCTAATA ACCAGTCCTA TAGAACAGCA

3351-400 GTAATAACTC AATTTAACTT AACCAGTGCC AAACTTCGAG AGACCGCAGT

3401-450 TTATTTTAGC AACTTAGTAG GTTATGAATT ATTGTTATTA CCAATATACG

3451-500 CACAAGTAGC AAATTTCAAT TTACTTTTAA TAAGAGATGG CCCTCATAAA

3501-550 TGCACAAGAA TGGTCTATGC ACGATCGTGT GACCAACTAT ATAACACTAT

3551-600 GGTGCAGTAC ACTAAAGAAT ATATTGCACA TAGCATTACA TGGTATAATA

3601-650 AAGGTTTAGA TGTACTTAGA AATAAATCTA ATGGACAATG GATTACGTTT

3651-700 AATGATTATA AAAGAGAGAT GACTATTCAA GTATTAGATA TACTCGCTCT

3701-750 TTTTGCCAGT TATGATCCAC GTCGATACCC TGCGGACAAA ATAGATAATA

3751-800 CGAAACTATC AAAAACAGAA TTTACAAGAG AGATTTATAC AGCTTTAGTA

3801-850 GAATCTCCTT CTAGTAAATC TATAGCAGCA CTGGAGGCAG CACTTACACG

3851-900 AGATGTTCAT TTATTCACTT GGCTAAAGAG AGTAGATTTC TGGACCAATA

3901-950 CTATATATCA AGATTTAAGA TTTTTATCTG CCAATAAAAT TGGGTTTTCA

3951-000 TATACAAATT CTTCTGCAAT GCAAGAAAGT GGAATTTATG GAAGTTCTGG

4001-050 TTTGGTTCAA ATCTATCTCA TCAAATTCAA CTTAATTCTA ATTGTTATAA

4051-100 AACTTCTATC ACAGATACTA GCTCCCCCTC TAATCGAGTT ACAAAAATGG

4101-150 ATTTCTACAA AATTTGATGG TACTCTTGCC TCTTATAATT CAAATATAAC

4151-200 ACCAACTCCT GAAGGTTTAA GGACCACATT TTTTGGATTT TCAACAAATG

4201-250 AGAACACACC TAATCAACCA ACTGTAAATG ATTATACGCA TATTTTAAGC

4251-300 TATATAAAAA CTGATGTTAT AGATTATAAC AGTAACAGGG TTTCATTTGC

4301-350 TTGGACACAT AACATTGTTG ACCCTAATAA TCAAATATAC ACAGATGCTA

4351-400 TCACACAAGT TCCGGCCGTA AAATCTAACT TCTTGAATGC AACAGCTAGA

# FIG. 7-3

```
4401-450 GTAATCAAGG GA  .TGGTCA TACAGGGGGG GATCTAGTTG CTCTTACAAG

4451-500 CAATGGTACT CTATCGGGAG GCAGAATGGA GATTCAATGT AAAACAAGTA

4501-550 TTTTTAATGA TCCTACAAGA AGTTACGGAT TACGCATACG TTATGCTGCA

4551-600 AATAGTCCAA TTGTGATTGA ATGTGATCAT ATGTATTACA AGGAGTTTCT

4601-650 AGAGGAACAA CGATTAGTAC AGAACTACGT TTCAAGACCT AATAATATAA

4651-700 TACCTACAGA TTTAAAATAT GAAGAGTTTA GATACAAAGA TCCTAATGAT

4701-750 GCAATTGTAC CGATGAGATT ATCTTCTAAT CAACTGATAA CTATAGCTAT

4751-800 TCAACCATTA AACATGACTT CAAATAATCA AGTGATTATT GACAGAATCG

4801-850 AAATTATTCC AATCACTCAA TCTGTATTAG ATGAGACAGA GAACCAAAAT

4851-900 TTAGAATCAG AACGAGAAGT TGTGAATGCA CTGTTTACAA ATGACGCGAA

4901-950 AGATGCATTA AACATTGGAA CGACAGATTA TGACATAGAT CAAGCCGCAA

4951-000 ATCTTGTGGA ATGTATTTCT GAAGGAATTA TATCCAAAGA AAAAATGCTC

5001-050 TTATTAGATG AAGTTAAAAA TGCGAAACAA CTTAGTCAAT CTCGAAATGT

5051-100 ACTTCAAAAC GGGGATTTTG AATCGCGTAC GCTTGGTTGG ACAACAAGTG

5101-150 ATAATATCAC AATTCAAGAA GATGATCCTA TTTTTAAAGG GCATTACCTT

5151-200 CATATGTCTG GGGCGAGAGA CATTGATGGT ACGATATTTC CGACCTATAT

5201-250 ATTCCAAAAA ATTGATGAAT CAAAATTAAA ACCGTATACA CGTTACCTAG

5251-300 TAAGGGGATT TGTAGGAAGT AGTAAAGATG TAGAACTAGT GGTTTCACGC

5301-350 TATGGGGAAG AAATTGATGC CATCATGAAT GTTCCAGCTG ATTTAAACTA

5351-400 TCTGTATCCT TCTACCTTTG ATTGTGAAGG GCTAATCGTT GTGAGCCTCC

5401-450 GCTGTGCCGC TAACATTTGG GACACTTCTG ATATGTTGTA TTCATGCCAA

5451-500 TATGATACAG GGAAAAAGCA TGTCGTATGT CAGGATTCCC ATCAATTTAG

5501-550 TTTCACTATT GATACAGGGG CATTAGATAC AAATGAAAAT ATAGGGGTTT

5551-600 GGGTCATGTT TAAAATATCT TCTCCAGATG GATACGCATC ATTAGATAAT

5601-650 TTAGAAGTAA TTGAAAGAGG GCCAATAGAT GGGGAAGCAC TGTCACGCGT

5651-700 GAAACACATG GAGAAGAAAT GGAACGATCA AATGGAAGCA AAACGTTCGG

5701-750 AAACACAACA AGCATATGAT GTAGCGAAAC AAGCCATTGA TGCTTTATTC

5751-800 ACAAATGTAC AAGATGAGGC TTTACAGTTT GATACGACAC TCGCTCAAAT

5801-850 TCAGTACGCT GAGTATTTGG TACAATCGAT TCCATATGTG TACAATGATT

5851-900 GGTTGTCAGA TGTTCCAGGT ATGAATTATG ATATCTATGT AGAGTTCGAT

5901-950 GCACGAGTGG CACAAGCGCG TTATTTGTAT GATACAAGAA ATATTATTAA
```

## FIG. 7-4

II / 16

```
5951-000  AAATGTTGAT  TTTACACAAG  GGGTAATGGG  GTGGCATGTA  ACTGGAAATG
60C1-050   CAGACGTACA  ACAAATAGAT  GGTGTTTCTG  TATTGGTTCT  ATCTAATTGG
6051-100   AGTGCTGGCG  TATCTCAAAT  GTCCATCTCC  AACTATAATC  ATGGGTATGT
6101-150   CTTACGTGTT  ATTGCCAAAA  AGAAGGACCT  GGAAATGGGT  ATGTCACGCT
6151-200   TATGGATTGT  GAGGAGAATC  AAGGAAAAAT  TAGCGTTTAC  GTCTTGTGAA
6201-250   GAAGGATATA  TTACGAAGAC  AGTAGATGTA  TTCCCAGATA  CAGCATTCGT
6251-300   GTCACGAATT  GAGATAGGCG  AAACCGAAGG  TTCGTTTTAT  ATCGAAAGCA
6301-350   TTGAAGTTAA  TTTGCATGAA  CGAGTGATTA  ATAAAAAATA  CCTAAAGCTT
6351-400   ATTAAACACT  GGAGAAAGTT  TTCTCCATGG  TTTTTAATTT  CTGCATTTAT
6401-450   TAATTTCTGG  TACAAAAAAT  ATATAGAAAA  CATAAAAAAT  AGATATCTAG
6451-500   AAAATCAATC  TCTTTTTCCA  AAGTTTGTTT  TTTAAATTTA  GCTGTCTCAA
6501-550   TATGTTTACG  GTCACAGCCA  CGTTCACCAC  GCTTCAACTC  AAAACCCTGT
6551-600   TTTTTCATAT  GCTCGGGGAA  TTTATCTTGT  AGCCATAACA  GTTCTTGACG
6601-650   ATTAAACACA  TTTTTTCCTT  GCAGTTTTCC  ATCACGCATA  GGCACAACAC
6651-700   CTAAATGCAT  GTGAGGGGTT  TGCTCATCAT  TATGAACTGT  TGCATAAGCA
6701-750   TATTTTGCTT  GCCATATCGT  TCGGAAAATA  ATTTATAACT  TTCCTCAAAA
6751-800   AATCGTTTTT  TGTTCTCCTG  GATCCAGTTG  CTCAAAAAAA  TCTCGGTCAG
6801-850   ATGTTACTAG  CAACTCATTT  ACAAGAACCG  CCATTCTTTC  CTCGTTTTTC
6851-900   TTGTACCTGT  TTTTTGTGAT  TCAATAATTT  CTTTGACACG  TTCGTTGTAA
6901-950   TCAATATTTT  TATCATTTTT  CAAATCCATT  AATTTTCACG  TGTTCGCTCA
6951-000   TGGTCAATAT  CATCATTCGT  TCTACTTTTT  CGGCTTCTCT  TTGATTATGG
7001-050   AAATTGCATG  CCTTTTAGTC  CAGCTGATTT  CACTTTTTGC  ATTCTACAAA
7051-100   CTGCATAACT  CATATGTAAA  TCGCTCCTTT  TTAGGTGGCA  CAAATGTCAG
7101-150   GCATTTTCGC  TCTTTCCGGC  AACCTTCCAA  GTAAAGTATA  ACACACTATA
7151-200   CTTTATATTC  ATAAAGTGTG  TGCTCTGCGA  GGCTGTCGGC  AGTGCCGACC
7201-250   AAAACCATAA  AACCTTTAAG  ACCTTTCTTT  TTTTTACGAG  AAAAAAGAAA
7251-300   CAAAAAAACC  TGCCCTCTGC  CACCTCAGCA  AAGGGGGGTT  TTGCTCTCGT
7301-350   GCTCGTTTAA  AAATCAGCAA  GGGACAGGTA  GTATTTTTTG  AGAAGATCAC
7351-400   TCAAAAAATC  TCCACCTTTA  AACCCTTGCC  AATTTTTATT  TTGTCCGTTT
7401-450   TGTCTAGCTT  ACCGAAAGCC  AGACTCAGCA  AGAATAAAAT  TTTTATTGTC
```

## FIG. 7-5

12/16

```
7451-500  TTTCGGTTTT  CTAGTGTAAC  GGACAAAACC  ACTCAAAAAT  AAAAAAGATA

7501-550  CAAGAGAGGT  CTCTCGTATC  TTTTATTCAG  CAATCGCGCC  CTTTAATGGA

7551-600  ACAAAAAGAT  TTGAATTCCT  GTTATAAAAA  AAGGATCAAT  TTTGAACTCT

7601-650  CTCCCAAAGT  TGATCCCTTA  ACGATTTAGA  AATCCCTTTG  AGAATGTTTA

7651-700  TATACATTCA  AGGTAACCAG  CCAACTAATG  ACAATGATTC  CTGAAAAAAG

7701-750  TAATAACAAA  TTACTATACA  GATAAGTTGA  CTGATCACTT  CCATAGGTAA

7751-800  CAACCTTTGA  TCAGTAAGGG  TATGGATAAT  AAACCAACCT  ACATTGCAAT

7801-850  AACCTGTTCC  CTCTGATTAA  AAAGCTGGTA  AAGTTAAGCA  AACTCATTCC

7851-900  AGCACCAGCT  TCCTGCTGTT  TCAAGCTACT  TGAACAATTG  TTGATATAAC

7901-950  TGTTTTGGTC  AACGAAAGCC  CACCTAAAAC  AAATACGATT  ATAATTGTCA

7951-000  TGAACCATGA  TGTTGTTTCT  AAAAGAAAGG  AAGCAGTTAA  AAAGCTAACA

8001-050  GAAAGAAATG  TAACTCCGAT  GTTTAACACG  TATAAAGGAC  CTCTTCTATC

8051-100  AACAAGTATC  CCACCAATGT  AGCCGAAAAT  AATGACACTC  ATTGTTCCAG

8101-150  GGAAAATAAT  TACACTTCCG  ATTTCGGCAG  TACTTAGCTG  GTGAACATCT

8151-200  TTCATCATAT  AACGAACCAT  AGAGACAAAC  CCTGCTACTG  TTCCAAATAT

8201-250  AATTCCCCCA  CAAAGAACTC  CAATCATAAA  AGGTATATTT  TTCCCTAATC

8251-300  CGGGACATAC  AAAAGGATCT  GTTACTTTCC  TGATATGTTT  TACAAATATC

8301-350  AGGAATGACA  GCACGCTAAC  GATAAGAAAA  GAAATGCTAT  ATGATGTTGT

8351-400  AAACAACATA  AAAAATACAA  TGCCTACAGA  CATTAGTATA  ATTCCTTTGA

8401-450  TATCAAAATG  ACCTTTTATC  CTTACTTCTT  TCTTTAATAA  TTTCATAAGA

8451-500  AACGGAACAG  TGATAATTGT  TATCATAGGA  ATGAGTAGAA  GATAGGACCA

8501-550  ATGAATATAA  TGGGCTATCA  TTCCACCAAT  CGCTGGACCG  ACTCCTTCTC

8551-600  CCATGGCTAC  TATCGATCCA  ATAAGACCAA  ATGCTTTACC  CCTATTTTCC

8601-650  TTTGGAATAT  AGCGCGCAAC  TACAACCATT  ACGAGTGCTG  GAAATGCAGC

8651-700  TGTTCCAATG  GAAAAGGTTA  ACATAAAGGC  TGTGTTCACC  CAGTTTGTAC

8701-750  TCGCAGGTGG  TTTATTAAAA  TCATTGCAAT  ATCACGTAAT  GAGACGTTCA

8751-800  AAACCATTTC  ATTTAATACG  CTAAAAAAGA  TAAAATGCAA  AGCCAAATTA

8801-850  AAATTTGGTT  GTGTCGTAAA  TTCGATTGTG  AATAGGATGT  ATTCACATTT

8851-900  CACCCTCCAA  TAATGAGGGC  AGACGTAGTT  TATAGGGTTA  ATGATACGCT

8901-950  TCCCTCTTTT  AATTGAACCC  TGTTACATTC  ATTACACTTC  ATAATTAATT

8951-000  CCTCCTAAAC  TTGATTAAAA  CATTTTACCA  CATATAAACT  AAGTTTTAAA
```

FIG. 7-6

13 176

```
9001-050  TTCAGTATTT CATCACTTAT ACAACAATAT GGCCCGTTTG TTGAACTACT
9051-100  CTTTAATAAA ATAATTTTTC CGTTCCCAAT TCCACATTGC AATAATAGAA
9101-150  AATCCATCTT CATCGGCTTT TTCGTCATCA TCTGTATGAA TCAAATCGCC
9151-200  TTCTTCTGTG TCATCAAGGT TTAATTTTTT ATGTATTTCT TTTAACAAAC
9201-250  CACCATAGGA GATTAACCTT TTACGGTGTA AACCTTCCTC CAAATCAGAC
9251-300  AAACGTTTCA AATTCTTTTC TTCATCATCG GTCATAAAAT CCGTATCTTT
9301-350  ACAGGATATT TTGCAGTTTC GTCAATTGCC GATTGTATAT CCGATTTATA
9351-400  TTTATTTTTC GGTCGAATCA TTTGAACTTT TACATTTGGA TCATAGTCTA
9401-450  ATTTCATTGC CTTTTTCCAA AATTGAATCC ATTGTTTTTG ATTCACGTAG
9451-500  TTTTCTGTAT TCTTAAAATA AGTTGGTTCC ACACATACCA ATACATGCAT
9501-550  GTGCTGATTA TAAGAATTAT CTTTATTATT TATTGCTCAC TTCCGTTGCA
9551-600  CGCATAAAAC CAACAAGATT TTTATTAATT TTTTTATATT GCATCATTCG
9601-650  GCGAAATCCT TGAGCCATAT CTGACAAACT CTTATTTAAT TCTTCGCCAT
9651-700  CATAAACATT TTTAACTGTT AATGTGAGAA ACAACCAACG AACATGTTGG
9701-750  CTTTTGTTTA ATAACTTCAG CAACAACCTT TTGTGACTGA AATGCCAATG
9751-800  TTTCATTAGC TCTCCTCCAG TTGCACATTG GACAAAGCCT GGATTTACAA
9801-850  AACCACACTC GATACAACTT TCTTTCGCCT GTTTCACGAT TTTGTTTATA
9851-900  CTCTAATATT TCAGCACAAT CTTTTACTCT TTCAGCCTTT TTAAATTCAA
9901-950  GAATATGCAG AAGTTCAAAG TAATCAACAT TAGCGATTTT CTTTTCTCTC
9951-000  CATGGTCTCA CTTTTCCACT TTTTGTCTTG TCCACTAAAA CCCTTGATTT
10001-050 TTCATCTGAA TAAATGCTAC TATTAGGACA CATAATATTA AAAGAAACCC
10051-100 CCATCTATTT AGTTATTTGT TTGGTCACTT ATAACTTTAA CAGATGGGGT
10101-150 TTTTCTGTGC AACCAATTTT AAGGGTTTTC AATACTTTAA AACACATACA
10151-200 TACCAACACT TCAACGCACC TTTCAGCAAC TAAAATAAAA ATGACGTTAT
10201-250 TTCTATATGT ATCAAGATAA GAAAGAACAA GTTCAAAACC ATCAAAAAAA
10251-300 GAACCTTTTC AGGTGCTTTT TTTATTTTAT AAACTCATTC CCTGATCTCG
10301-350 ACTTCGTTCT TTTTTTACTC TCGGATTATC ACATTGTTCA AATTCGTTCT
10351-400 TTTTAGGTTC TAAATCGTGT TTTTCTTGGA ATTGTGCTGT TTTATCCTTT
10401-450 ACCTTGTCTA CAAACCCCTT AAAAACGTTT TTAAAGCTTT TAAGCCGTCT
10451-500 GTAACGTTCC TTAAGGAATT CCTTAGTGCT TTCATAGATT AAACTCACAA
```

# FIG. 7-7

141 16

10501-550 CTACGCTTTT AAATCGCTTA TTTAGACTTT AAAGACTTGT TTTCTTCAAG

10551-600 CAACTCATTA TAATCATTTA CATTTTCATT AAATCGCTCT ACAAGACCAC

10601-650 TATATTTTTC TTTAACTTGC CCATGTTCTT TACTTAATTT TTTATATTCT

10651-700 CTCGCCATAT CAGTACTCAT GAGATTTCTA ACATGCTGTT TTAACCTATC

10701-750 GTTATCTCTC GCAGCAGTCA CTAAGTTTTT ATAATCACGC TCCGATATAA

10751-800 CAACATTTTT TGGTTGGTTT CTTTTCTGTT TTCATTATTT CTTTTCCCAA

10801-850 ACCAAACATG GACTTTTCAC CGTTGGCACT TCAACACTTT TCATGTGTCG

10851-900 TTTCGCTGGT ACTTCTAAAT CTGATTTAAC TTTATCGCTA TAAGCAGTCC

10901-935 ATTCATCTTT TTTAACTGCT AAATTTTTTT CTAGA

# FIG. 7-8

TATATATTCCAAAAAATTGATGAATCAAAATTAAAACCGTATACACGTTACCTAGTAAGGGGATTTGTAGGAAGTAGTAAAGATGTAGAACTAGTGGTTTCACGCTATGGGGAAGAAATT
TyrIlePheGlnLysIleAspGluSerLysLeuLysProTyrThrArgTyrLeuValArgGlyPheValGlySerSerLysAspValGluLeuValValSerArgTyrGlyGluGluIle

GATGCCATCATGAATGTTCCAGCTGATTTAAACTATCTGTATCCTTCTACCTTTGATTGTGAAGGGCTAATCGTTGTGAGCGTCCGCTGTGCCGCTAACATTTGGGACACTTCTGATATG
AspAlaIleMetAsnValProAlaAspLeuAsnTyrLeuTyrProSerThrPheAspCysGluGlyLeuIleValValSerValArgCysAlaAlaAsnIleTrpAspThrSerAspMet

TTGTATTCATGCCAATATGATACAGGGAAAAAGCATGTCGTATGTCAGGATTCCCATCAATTTAGTTTCACTATTGATACAGGGGCATTAGATACAAATGAAAATATAGGGGTTTGGGTC
LeuTyrSerCysGlnTyrAspThrGlyLysLysHisValValCysGlnAspSerHisGlnPheSerPheThrIleAspThrGlyAlaLeuAspThrAsnGluAsnIleGlyValTrpVal

ATGTTTAAAATATCTTCTCCAGATGGATACGCCATCATTAGATAATTTTAGGAAGTAATTGAAAGAGGGGCCAATAGATGGGGAAGCACTGTCACGCGTGAAACACATGGAGAAGAAATGGAAC
MetPheLysIleSerSerProAspGlyTyrAlaSerLeuAspAsnLeuGluValIleGluArgGlyProIleAspGlyGluAlaLeuSerArgValLysHisMetGluLysLysTrpAsn

GATCAAATGGAAGCAAAACGTTCGGAAACACAACAAGCATATGATGTAGCGAAACAAGCCATTGATGCTTTATTCACAAATGTACAAGATGAGGCTTTACAGTTTGATACGACACTCGCT
AspGlnMetGluAlaLysArgSerGluThrGlnGlnAlaTyrAspValAlaAlaLysGlnAlaIleAspAlaLeuPheThrAsnValGlnAspGluAlaLeuGlnPheAspThrThrLeuAla

CAAATTCAGTACGCTGAGTATTTGGTACAATCGATTCCATATGTGTACAATGATTGGTTGTCAGATGTTCCAGGTATGAATTATGATATCTATGTAGAGTTGGATGCACGAGTGGCACAA
GlnIleGlnTyrAlaGluTyrLeuValGlnSerIleProTyrValTyrAsnAspTrpLeuSerAspValProGlyMetAsnTyrAspIleTyrValGluLeuAspAlaArgValAlaGln

GCGCGTTATTTGTATGATACAAGAAATATTATTAAAAATGTTGATTTTACACAAGGGGTAATGGGGTGGCATGTAACTGGAAATGCAGACGTACAACAAATAGATGGTGTTTCTGTATTG
AlaArgTyrLeuTyrAspThrArgAsnIleIleLysAsnValAspPheThrGlnGlyValMetGlyTrpHisValThrGlyAsnAlaAspValGlnGlnIleAspGlyValSerValLeu

GTTCTATCTAATTGGAGTGCTGGCGTATCTCAAATGTCCATCTCCAACTATAATCATGGGTATGTCTTACGTGTTATTGCCAAAAAGAAGGACCTGGAAATGGGTATGTCACGCTTATGG
ValLeuSerAsnTrpSerAlaGlyValSerGlnMetSerIleSerAsnTyrAsnHisGlyTyrValLeuArgValIleAlaLysLysLysAspLeuGluMetGlyMetSerArgLeuTrp

ATTGTGAGGAGAATCAAGGAAAAATTAGCGCGTTTACGTCTTGTGAAGAAGCGGATATATTACGAAGACAGTAGATGTATTCCCAGATACAGCATTCGTGTCACGAATTGAGATAGGCGAAACC
IleValArgArgIleLysGluLysLeuAlaPheThrSerCysGluGlnGlyTyrIleThrLysThrAlaIleAspValPheProAspThrAlaPheValSerArgIleGluIleGlyGluThr

GAAGGTTCGTTTTTATATCGAAAGCATTGAAGTTAATTTGCATGAACGAGTGATTAATAAAAAAATACCTAAAGCTTATTAAACACTGGAGAAAGTTTTCTCCATGGTTTTTAATTTCTGCA
GluGlySerPheTyrIleGluSerIleGlyGluValAsnLeuHisGluArgValIleAsnLysLysTyrLeuLysLeuIleLysHisTrpArgLysPheSerProTrpPheLeuIleSerAla

TTTATTAATTTCTGGTACAAAAAAATATATAGAAAACATAAAAAATAGATATCTAGA
PheIleAsnPheTrpTyrLysLysTyrIleGluAsnIleLysAsnArgTyrLeu

## FIG. 8-2

0195285

0195285

```
                    10                    30                    50                    70                    90                   110
ATGAATTCAGGCTATCCGTTAGCGAATGACTTACAAGGGTCAATGGTCTAGCCATGTGTGAAAATAACAACCAACAGTATGGCGTAATCCAGCTGCCATT
MetAsnSerGluTyrProLeuAlaAsnAspLeuGlnGlySerMetLysTrpLeuAlaMetCysGluAsnAsnGlnGlnTyrGlyValAsnProAlaAlaIle
                   130                   150                   170                   190                   210                   230
AATTCTTCAGTTAGTACCCCTTTAAAAGATGCTGGAGCTATCCTTAAAATTGTAAACCCACCTGCAGGAATCTCTGTCTTAAGCGTACTTAGCGCGGTGCTTCCTATTCTTTGGCCGACT
AsnSerSerValSerThrAlaLeuLysAspAlaGlyAlaIleLeuLysIleValAsnProProAlaGlySerValLeuSerValLeuSerAlaValLeuProIleLeuTrpProThr
                   250                   270                   290                   310                   330                   350
AATACTCCAACGCCTGAAAGAGCCTGAAATCATTTGGAATCTTATTGATCAAACTGTAACAGCTTATGTATGCAACAGATGCAAAATGCAAAATGACGGTTGTGAAA
AsnThrProThrProGluArgValTrpAsnAspPheMetThrArgAsnThrGlyAsnLeuIleAspGlnThrValThrAlaTyrValAlaValCysMetThrValValLys
                   370                   390                   410                   430                   450                   470
AATACTTATAGATCAATATAACATAAATTTAACACTTGGAAGAAGAGACCCTAATAACCAGTCCTATAGAACCAATCAATTTAACTTAACCAGTGCCAAACTTCCAGAGACC
AsnThrTyrLeuAspGlnTyrThrThrLysPheAsnThrTrpLysArgGluProAsnAsnGlnSerTyrArgThrAlaValIleThrGlnPheAsnLeuThrSerAlaLysLeuArgGluThr
                   490                   510                   530                   550                   570                   590
GCAGTTATTTAGGAACTAGTAGGTATATGAATAATTGTTATTACCAATATACGCCACAGTAGCAAATTTACTTTAATAAGAGAGATGGCCCTCATAAATGCACAAGAAATGTC
AlaValIleTyrPheSerAlaGlnTyrThrLysPheAsnLeuLeuLeuProIleTyrAlaGlnValAlaAsnPheAsnLeuLeuIleArgAspGlyProHisLysCysThrArgMetVal
                   610                   630                   650                   670                   690                   710
TATGCACGATCGTGTGACCAACTATCATACCACTATGGTCAGTACCATTACCATCAGCTAAAGAAGAATATATTGCCACATTAGAAGGCTTTAGATGTACTTAGAAAATAAATCTAATCGA
TyrAlaArgSerCysAspThrThrIleHisProThrMetValGlnTyrHisTyrHisGlnLeuLysLysAsnIleLeuProHisLeuGluGlyPheArgCysThrLeuGluAsnLysSerAsnArg
                   730                   750                   770                   790                   810                   830
CAATGGATTACGTTTAATAAAAGAGAGATGACTATTTCAAGTATTCCAGTATACTCGCTCTTTTGCCAGTTGCACGTCGATACCCTGCCGACAAAAATAGATATAACCGAAA
GlnTrpIleThrPheAsnLysArgGluMetThrIleSerSerIleProValTyrSerLeuPheAlaSerCysThrSerIleProAlaAspLysAsnArgTyrAsnArgLys
                   850                   870                   890                   910                   930                   950
CTATCAAAAACAGAATTTACAAGAGATTTATACAGCTTAGTAGAATCTCCTTCTAGTAAATCTATAGCTTAGTGTTTTCATATAAATTGGGTTTCATTATTATTCACTTGGCTA
LeuSerLysThrGluPheThrArgAspLeuTyrSerLeuValGluSerProSerSerLysSerIleAlaLeuValPheSerIleLysLeuGlyPheHisIleLeuPheThrTrpLeu
                   970                   990                  1010                  1030                  1050                  1070
AAGAGAGTAGAATTCTCGACCAATACTATATATCAAGATTTAAGATTTTATCTGCCAATAAATTCTTCTATCACAGATCTAGCTCAGCTTTCATATACAAATTCTTCTCCAATGCAAGAAAGTGGAATTTATGGAAGT
LysArgValGlyValAspPheTrpThrAsnThrIleTyrGlnAspLeuArgPheTyrLeuSerAlaAsnAsnSerSerIleThrAspLeuAlaGlnLeuSerTyrThrAsnSerSerProMetGlnGluSerGlyIleTyrGlySer
                  1090                  1110                  1130                  1150                  1170                  1190
TCTGGTTTGGTTCAAATCTATCTCATCAAGTTACTTTACTTCAACTTGTTTATGTTATTATAGAAAACTTCTAATGTTGTTACTCAGAGATACTAGCTCCCCTCTAATGGATTCTACAGAAAAATGGATTTTCTACAAAATTCATGTGGAGATTCTACCTAGCTGAAGT
SerGlyLeuValGlnIleTyrLeuIleLysLeuLeuTyrLeuGlnLeuValTyrValIleIleGluLysLeuLeuMetLeuLeuLeuArgAspThrSerSerProLeuMetAspSerThrGluLysTrpIleSerLeuGlnAsnSerCysGlyAspSerThrAlaGluVal
                  1210                  1230                  1250                  1270                  1290                  1310
GATGGTACTCTTGCCTCTTATAATTCAAATATAACACCAACTCGTCGAAGGTTCAACTAACGTAACTGAAGGGTTTCATTGGGTTTCGGGTTAGCGGGATACTAGCTCCAACTGTAAATGATTAT
AspGlyThrLeuAlaSerTyrAsnSerAsnIleThrProThrArgArgArgPhePheGlyPheGlyGlyLeuAlaGlyTyrThrAlaProThrValAsnAspTyr
                  1330                  1350                  1370                  1390                  1410                  1430
ACGGATATTTTTAAGCTATATAAAAACTGATGTTATAGATATATGAACAGTAACAGTAGAAGGCTAGGTGCCTTCTTACCAGTGTCCTCTTACAAGCAATGTACTGTTCTTACAAGGACTATAACCTATCCA
ThrHisIleLeuSerTyrIleLeuSerThrAspValIleAspIleTrpThrValThrValGluGlyLeuGlyAlaPheLeuProValSerSerTyrLysGlnCysThrValLeuThrArgThrIleThr
                  1450                  1470                  1490                  1510                  1530                  1550
CAAGTTCCGGCCGTAAAATCTAACTTCTTGAATGCAAGCTAGAGTAATCAAGGGACCTGGTCATCAGGTAATCCAAATAGTGTACTGTACTCATCGGGCAGGCAGA
GlnValProAlaValLysSerAsnPheLeuAsnAlaSerTyrAlaArgValIleLysGlyProGlyHisThrGlyGlnValIleGlnIleValTyrTyrLeuSerGlyGlyArg
                  1570                  1590                  1610                  1630                  1650                  1670
ATGGAGATTCAATGTAAAAACAAGTATTTTTAATGATCCTACAAGAAAGTTACGGATTACCGCCATACGTTATGCTGCAAATAGTCCAAATAGTCCAAATAGTGATGCGTATCGTATTATTACAAGGAG
MetGluIleGlnCysLysThrSerIleLeuPheAsnAspProThrArgSerTyrGlyLeuArgIleArgTyrAlaAlaAsnSerProIleGluGluValIleGluGlnSerMetTyrTyrLysGlu
                  1690                  1710                  1730                  1750                  1770                  1790
TTTCTAGAGGGAACAAGCAGCAAGGACGTAGTACAGAACTACCTTCAAGGACCTAATAATAATATTCCTACAGATTTAAAAATGAAGAGTTAGAAACAAAGATCCTAATGAATCTTAGAGTACAAGGTCAATTGTACCGGATG
PheLeuGluGlyThrSerSerLysAspValValGlnAsnTyrLeuGlnGlyProAsnAsnAsnIleProThrAspLeuLysMetLysSerTyrGluGluPheArgTyrLysAspProAsnAspAlaIleValProMet
                  1810                  1830                  1850                  1870                  1890                  1910
AGATATCTTCTAATCAACTGATAACTAGCTATTCAACCATTAAAACATAAAGCAAGTGATTATTGACAGAATCGAATTATTCCAATGACTCAATGTGTATTAGATGAG
ArgLeuSerSerAsnGlnLeuIleThrIleAlaIleGlnProLeuGluAsnMetThrSerAsnAsnGlnValIleIleAspArgArgIleGlnIleIleProIleThrGlnSerValLeuGluAspGlu
                  1930                  1950                  1970                  1990                  2010                  2030
ACAAGGGAACAAGCGAAGAGTTGTCGAATGCAGTCTTACCAATGACAGCCGCAAATGAACGACCGGAAATGGAACATTATTCCAATGACTCAATGTGTATTAGAAGGCCGCAAATCTT
ThrArgGluGlnAlaLysSerLeuSerAsnAlaValLeuProMetThrAlaAlaAsnGluArgProGluMetGluHisTyrSerAsnAspSerMetCysIleArgArgProGlnIleLeu
                  2050                  2070                  2090                  2110                  2130                  2150
GTCGAAATGTATTTTCGAAGGAATTATATCCAAAGAAAAAAATGTCTCTATTAGATGAATAACAACTTAGTCAATCTCGAAAATGGGGATTTTGAATCG
ValGlnAsnGlnIleAsnLeuGluGlyIleIleIleSerLysGluLysMetLeuLeuLeuLeuAspGluAsnAsnLeuSerGlnSerArgAsnValLeuGlnAsnGlyAspPheGluSer
                  2170                  2190                  2210                  2230                  2250                  2270
CGTACCCTTGGTTGGACAACAACATGAATAAATCACAATTCAAGAAAGATGATCCTATTTTTAAAGGGCAATTCCTATTACTTCCATATCTGTATTAGAACC
ArgThrLeuGlyTrpThrThrSerAsnAspAsnIleThrIleGlnGluIleGlnHisTyrLeuHisMetSerGlyAlaArgAspIleGlnThrIleProPheProThr
                  2290                  2310                  2330                  2350                  2370                  2390
```

*FIG. 8-1*